(19) Europäisches Patentamt
European Patent Office
Office européen des brevets

(11) EP 4 190 824 A1

(12) EUROPEAN PATENT APPLICATION
published in accordance with Art. 153(4) EPC

(43) Date of publication:
**07.06.2023 Bulletin 2023/23**

(21) Application number: **21850270.6**

(22) Date of filing: **30.07.2021**

(51) International Patent Classification (IPC):
***C08F 20/56*** *(2006.01)* ***A61K 47/58*** *(2017.01)*
***G01N 30/06*** *(2006.01)* ***G01N 30/88*** *(2006.01)*

(52) Cooperative Patent Classification (CPC):
**A61K 47/58; C08F 20/56; G01N 30/06; G01N 30/88**

(86) International application number:
**PCT/JP2021/028274**

(87) International publication number:
**WO 2022/025228 (03.02.2022 Gazette 2022/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.07.2020 JP 2020129825**

(71) Applicants:
- **Toray Industries, Inc.**
**Tokyo 103-8666 (JP)**
- **Biodynamic Research Foundation**
**Kumamoto-shi, Kumamoto 862-0954 (JP)**

(72) Inventors:
- **OKANO, Tsubasa**
**Kamakura-shi, Kanagawa 248-8555 (JP)**
- **UJIIE, Satoshi**
**Kamakura-shi, Kanagawa 248-8555 (JP)**

(74) Representative: **Kador & Partner Part mbB**
**Corneliusstraße 15**
**80469 München (DE)**

# (54) METHOD FOR EVALUATING DEGREE OF PURITY OF PHARMACEUTICAL SUBSTANCE CONTAINED IN COMPOSITE BODY, AND METHOD FOR PRODUCING COMPOSITE BODY

(57) Disclosed arc a method that enables evaluation of the purity of an active pharmaceutical ingredient contained in a complex, and a method of producing a complex in which the purity of the active pharmaceutical ingredient is not less than 95.0%. The purity evaluation method includes: a reaction step of reacting a complex with a nitrogen-containing nucleophile such as hydroxylamine in the presence of a protonic acid in a polar solvent; and an evaluation step of evaluating the purity of the reaction mixture obtained by the reaction step, by high-performance liquid chromatography. The method of producing a complex includes a reaction step of reacting an anthracycline drug with an *N*-(2-hydroxypropyl)methacrylamide polymer in the presence of a protonic acid in a polar solvent at not more than 10°C, to obtain the complex.

EP 4 190 824 A1

## Description

TECHNICAL FIELD

The present invention relates to a method of evaluating the purity of a drug contained in a complex, and a method of producing a complex.

BACKGROUND ART

A complex (hereinafter referred to as P-THP) composed of pirarubicin (hereinafter referred to as THP), which is an anthracycline drug, and an *N*-(2-hydroxypropyl)methacrylamide polymer, which is a biocompatible macromolecular compound, bound to each other through a hydrazone bond is a compound useful as an antitumor drug or an anticancer drug (Patent Documents 1 and 2).
As a method of producing P-THP, for example, a method comprising reacting THP with an *N*-(2-hydroxypropyl)methacrylamide polymer in the presence of acetic acid has been disclosed (Non-Patent Document 1).
Further, as a method of quantifying a complex composed of anthracycline drug doxorubicin (hereinafter referred to as DOX) and an *N*-(2-hydroxypropyl)methacrylamide polymer bound to each other through an oligopeptide, a method comprising adding hydrochloric acid to a mixture of the complex to carry out hydrolysis has been disclosed (Non-Patent Document 2).

PRIOR ART DOCUMENTS

[Patent Documents]

[Patent Document 1] JP 5904602 B
[Patent Document 2] WO 2017/191843

[Non-Patent Documents]

[Non-Patent Document 1] Etrych et al., European Journal of Pharmaceutical Sciences , 2017, vol. 106, pp. 10-19
[Non-Patent Document 2] Fraier et al., Journal of Pharmaceutical and Biomedical Analysis, 1995, vol. 13, pp. 625-633

SUMMARY OF THE INVENTION

PROBLEMS TO BE SOLVED BY THE INVENTION

In general, pharmaceuticals are subjected to strict purity evaluation for the purpose of quality confirmation. For example, according to the definition on the purity of DOX hydrochloride described in the Japanese Pharmacopoeia, DOX hydrochloride should show a titer of 98.0 to 108.0% depending on the quantification method. However, in complexes such as P-THP, an active pharmaceutical ingredient (API) is bound to a macromolecular compound (such as a polymer) or the like, and therefore direct evaluation of the purity of the active pharmaceutical ingredient using the complex is technically difficult. Thus, for evaluation of the purity of the active pharmaceutical ingredient, the active pharmaceutical ingredient needs to be released from the complex. However, for example, when THP is released from P-THP by the method described in Non-Patent Document 2, degradation of the THP into DOX and other degradation products occurs at the same time as the release of the THP, so that strict/accurate evaluation of the purity of the active pharmaceutical ingredient contained in the complex has so far been difficult (the purity of the active pharmaceutical ingredient contained in the complex, that is, the purity of the active pharmaceutical ingredient bound to the macromolecular compound, is hereinafter also referred to as "bound-drug purity"). When the later-described purity evaluation method of the present invention was carried out for evaluation of the bound-drug purity of P-THP produced by the method described in Non-Patent Document 1, the P-THP was found not to satisfy the THP purity standard (95.0% or higher) described in the Japanese Pharmacopoeia. Thus, it was newly discovered that P-TIIP produced by the conventional method has a problem in the bound-drug purity.
In view of this, an object of the present invention is to provide a method that enables evaluation of the purity of an active pharmaceutical ingredient contained in a complex. Another object of the present invention is to provide a

method of producing an anthracycline-drug-containing complex whose drug purity is not less than 95.0% as evaluated by the purity evaluation method of the present invention.

MEANS FOR SOLVING THE PROBLEMS

As a result of intensive study to solve the above problem, the present inventors discovered that evaluation of the bound-drug purity is possible by reacting a complex having a hydrazone bond with a certain nitrogen-containing nucleophile in the presence of a protonic acid in a polar solvent to convert the anthracycline drug into a stable drug equivalent, and that a highly pure complex can be produced by reacting an anthracycline drug with an *N*-(2-hydroxypropyl)methacrylamide polymer in the presence of a protonic acid in a polar solvent at not more than 10°C, thereby completing the present invention.

More specifically. the present invention provides the following:

[1] A method of evaluating the purity of a drug contained in a complex of General Formula (I):

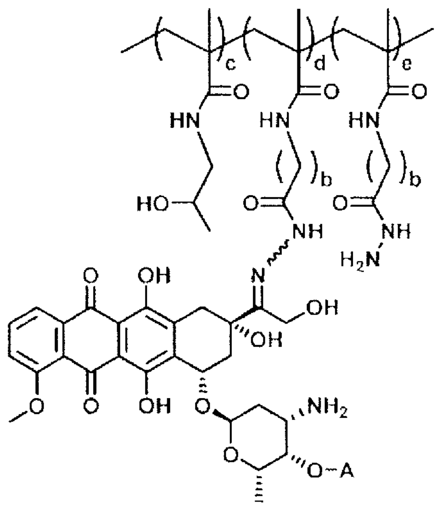

(I)

[wherein A is a hydrogen atom or (*R*)-tetrahydro-2*H*-pyran-2-yl; b, c, d, and e each independently are a positive integer; and the bond indicated by a wavy line represents that the complex has either *E* or *Z* configuration] or a pharmaceutically acceptable salt thereof, the method comprising:

a reaction step of reacting the complex of General Formula (I) or the pharmaceutically acceptable salt thereof with at least one nitrogen-containing nucleophile selected from the group consisting of hydroxylamine, *O*-alkylhydroxylamine, and carboxylic acid hydrazide in the presence of a protonic acid in a polar solvent; and

an evaluation step of evaluating the purity of the reaction mixture obtained by the reaction step, by high-performance liquid chromatography.

[2] The method according to [1], wherein

the polar solvent is an alcoholic solvent;

the nitrogen-containing nucleophile is at least one selected from the group consisting of hydroxylamine and carboxylic acid hydrazide; and

the protonic acid is a carboxylic acid.

[3] The method according to [1] or [2], wherein

the polar solvent is methanol;

the nitrogen-containing nucleophile is at least one selected from the group consisting of hydroxylamine, acetohydrazide, propanohydrazide, butyrohydrazide, and 3-methylbutanohydrazide; and

the protonic acid is acetic acid.

[4] The method according to any one of [1] to [3], wherein in the General Formula (I), b is an integer of 1 to 10; c is an integer of 30 to 500; d is an integer of 1 to 50; and e is an integer of 1 to 50.

[5] A complex of General Formula (I):

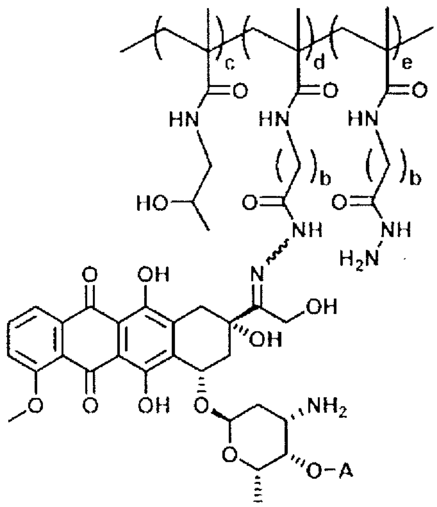

(I)

[wherein A is a hydrogen atom or (*R*)-tetrahydro-2*H*-pyran-2-yl; b, c, d, and e each independently are a positive integer; and the bond indicated by a wavy line represents that the complex has either *E* or *Z* configuration] or a pharmaceutically acceptable salt thereof, wherein the drug contained in the complex of General Formula (I) or the pharmaceutically acceptable salt thereof has a purity of not less than 95.0% as evaluated by the method according to any one of [1] to [4].

[6] The complex or the pharmaceutically acceptable salt thereof according to [5], wherein

A is (*R*)-tetrahydro-2*H*-pyran-2-yl; and
b is 5.

[7] A method of producing a complex of General Formula (I):

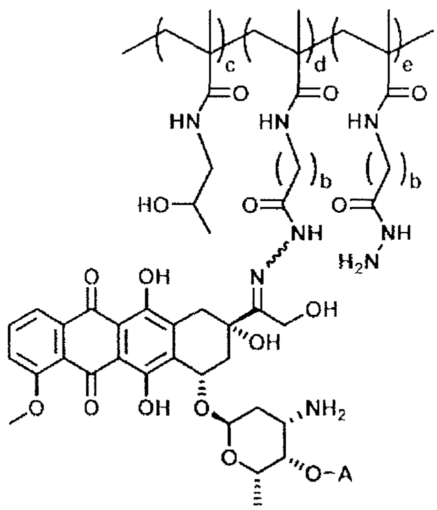

(I)

or a pharmaceutically acceptable salt thereof, the method comprising a reaction step of reacting an anthracycline drug of General Formula (II):

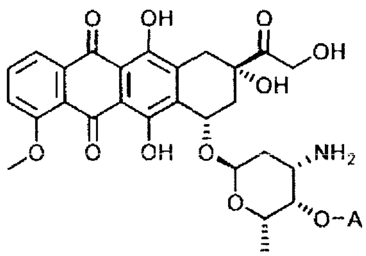

(II)

with an *N*-(2-hydroxypropyl)methacrylamide polymer of General Formula (III):

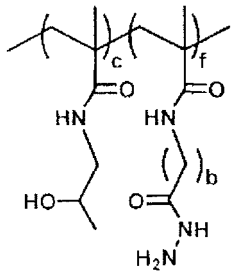

(III)

in the presence of a protonic acid in a polar solvent at not more than 10°C, to obtain the complex of General Formula (I) or the pharmaceutically acceptable salt thereof [0018]

[wherein in Formula (I) and Formula (II), A is a hydrogen atom or (*R*)-tetrahydro-2*H*-pyran-2-yl; in Formula (I) and Formula (III), b, c, d, e, and f each independently are a positive integer; and, in Formula (I), the bond indicated by a wavy line represents that the complex has cither *E* or *Z* configuration].

[8] The method according to [7], wherein

the polar solvent is methanol;
the protonic acid is acetic acid; and
the reaction temperature during the reaction step is -30°C to 10°C.

[9] The method according to [7] or [8], wherein in the General Formula (I) and the General Formula (III), b is an integer of 1 to 10; c is an integer of 30 to 500; d is an integer of 1 to 50; e is an integer of 1 to 50; and f is the sum of d and e.

MODE FOR CARRYING OUT THE INVENTION

1. Method of Evaluating Purity of Drug Contained in Complex

The method of evaluating the purity according to the present invention comprises:

a reaction step of reacting a complex of General Formula (I):

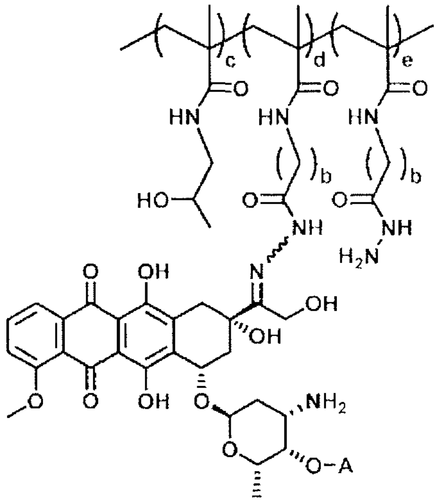

(I)

[wherein A is a hydrogen atom or (*R*)-tetrahydro-2*H*-pyran-2-yl; b, c, d, and e each independently are a positive integer; and the bond indicated by a wavy line represents that the complex has either *E* or *Z* configuration]
or a pharmaceutically acceptable salt thereof with a nitrogen-containing nucleophile selected from the group consisting of hydroxylamine, *O*-alkylhydroxylamine, and carboxylic acid hydrazide in the presence of a protonic acid in a polar solvent; and
an evaluation step of evaluating the purity of the reaction mixture obtained by the reaction step, by high-performance liquid chromatography.

In the present description and claims, when a wavy line is included in a chemical formula, the wavy line represents that the compound has either *E* or *Z* configuration, and the compound may be any of an E-isomer alone, a Z-isomer alone, or a mixture of the E-isomer and the Z-isomer.
From the viewpoint of the efficiency of transport by a transporter, A is preferably (*R*)-tetrahydro-2*H*-pyran-2-yl.
Although b, c, d, and e are not limited as long as they are positive integers, b is preferably an integer of 1 to 10 (for example, 5); c is preferably an integer of 30 to 500, especially preferably an integer of 50 to 500; d is preferably an integer of 1 to 50; and e is preferably an integer of 1 to 50.
The "complex" herein means an anthracycline-drug-containing complex in which an anthracycline drug of General Formula (11):

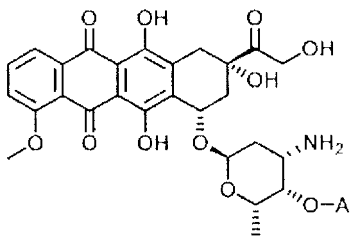

(II)

[wherein A is a hydrogen atom or (*R*)-tetrahydro-2*H*-pyran-2-yl]
is bound to an *N*-(2-hydroxypropyl)methacrylamide polymer of General Formula (III):

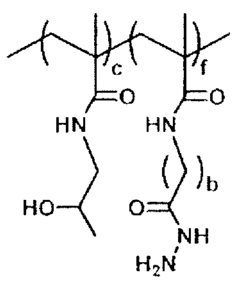

(III)

[wherein b, c, and f each independently are a positive integer]
through a hydrazone bond, and the anthracycline drug of General Formula (II) means the active component of the complex. The anthracycline drug of General Formula (II) means THP or DOX; the drug in which THP is bound to an *N*-(2-hydroxypropyl)methacrylamide polymer of General Formula (III) through a hydrazone bond means P-THP; and the drug in which DOX is bound to an *N*-(2-hydroxypropyl)methacrylamide polymer of General Formula (III) through a hydrazone bond means P-DOX.

In the present description, "a drug contained in a complex of General Formula (1) (hereinafter referred to as Complex (I)) or a pharmaceutically acceptable salt thereof" means an anthracycline drug contained in the complex or the pharmaceutically acceptable salt thereof.

In the present description, "bound-drug purity" means an estimated purity of the drug contained in Complex (I), and means the area percentage of the measurement target peak as determined based on a chromatogram obtained by high-performance liquid chromatography (hereinafter referred to as HPLC) according to the following method, wherein the total area of peaks excluding the blank peak is taken as 100%. The blank peak means the peak detected when methanol is measured under the following HPLC conditions. Although the measurement target peak varies depending on the type of the anthracycline drug and the type of the nitrogen-containing nucleophile, it means the peak of the anthracycline drug equivalent based on the molecular weight. In cases where separated peaks of isomers appear under the following analysis conditions, the sum of the area percentages of the peaks of the isomers is used as the area percentage of the measurement target peak. The following description is an example of a case using hydroxylamine as the nitrogen-containing nucleophile, methanol as the polar solvent, and acetic acid as the protonic acid. Conditions such as the amounts of their addition may be appropriately changed depending on the reagents used.

(1) Sample Preparation

To a solution of a complex (10 mg) in methanol (160 μL), 50% by weight aqueous hydroxylamine solution (32 μL) and acetic acid (27 μL) are added at 0°C, and the resulting mixture is stirred at 0°C for 25 hours. Methanol (450 μL) is added to the obtained reaction mixture (50 μL), to provide a measurement sample.

(2) HPLC

The measurement sample prepared as described above is subjected to analysis using HPLC under the following measurement conditions.

Detector: photodiode array detector (measurement wavelength: 488 nm)
Column: Scherzo SM-C18 (manufactured by Imtakt Corporation)
Column size: 150×4.6 mm (3 μm)
Column temperature: 30°C
Mobile phase:

Solution A, 5 mmol/L ammonium formate reagent solution / methanol mixed solution (95:5)
Solution B, methanol / water / formic acid mixed solution (90:10:0.1)
Developing condition:

A/B = 75/25 (minute 0 to 3)
A/B = 75/25 to 37/63 (minute 3 to 4; linear gradient)
A/B = 37/63 (minute 4 to 24)
A/B = 37/63 to 20/80 (minute 24 to 25; linear gradient)
A/B = 20/80 (minute 25 to 35)
A/B = 20/80 to 0/100 (minute 35 to 39; linear gradient)
A/B = 0/100 (minute 39 to 54)
A/B = 0/100 to 75/25 (minute 54 to 54.1; linear gradient)
A/B = 75/25 (minute 54.1 to 59.5)

Flow rate: 1.0 mL/minute
Injection volume: 20 μL
Sample cooler temperature: 4°C

In the *O*-alkylhydroxylamine in the method of evaluating the purity according to the present invention, the alkyl

group preferably has 1 to 4 carbon atoms. Preferred examples of the *O*-alkylhydroxylamine include *O*-methylhydroxylamine, *O*-ethylhydroxylamine, and *O*-propylhydroxylamine. From the viewpoint of suppressing side reactions, *O*-methylhydroxylamine and *O*-ethylhydroxylamine are preferred. *O*-Methylhydroxylamine is more preferred. The *O*-alkylhydroxylamine used in the present invention may be a single compound, or may be a combination of two or more kinds of compounds.

Examples of the carboxylic acid hydrazide in the method of evaluating the purity according to the present invention include formhydrazide, acetohydrazide, propanohydrazide, butyrohydrazide, 3-methylbutanohydrazide, 2,2-dimethylpropanohydrazide, cyclohexanecarbohydrazide, and adamantane-1-carbohydrazide. From the viewpoint of suppressing side reactions, at least one selected from the group consisting of acetohydrazide, propanohydrazide, butyrohydrazide, and 3-methylbutanohydrazide is preferred. Acetohydrazide is more preferred. The carboxylic acid hydrazide used in the present invention may be a single compound, or may be a combination of two or more kinds of compounds.

The nitrogen-containing nucleophile is preferably at least one selected from the group consisting of hydroxylamine and carboxylic acid hydrazide, more preferably at least one selected from the group consisting of hydroxylamine, acetohydrazide, propanohydrazide, butyrohydrazide, and 3-methylbutanohydrazide, still more preferably at least one selected from the group consisting of hydroxylamine and acetohydrazide.

In cases where the drug is released from the complex using the nitrogen-containing nucleophile described above, the drug is released after conversion to a corresponding drug equivalent. Since the released drug equivalent has excellent stability under the reaction conditions, the method is preferred as a method of evaluating the bound-drug purity.

The "drug equivalent" herein means an anthracycline drug equivalent of the following General Formula (IV) or General Formula (V).

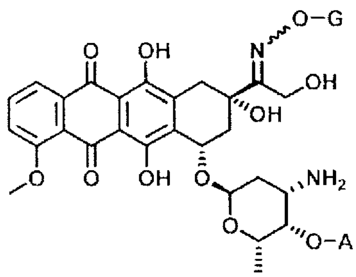

(IV)

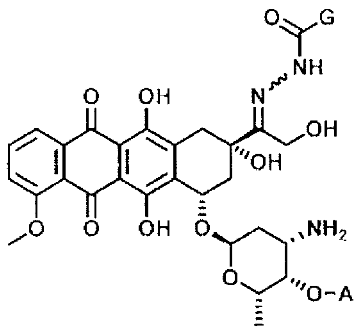

(V)

[In Formula (IV) and Formula (V), A is a hydrogen atom or (*R*)-tetrahydro-2*H*-pyran-2-yl; G is a hydrogen atom or alkyl; and the bond indicated by a wavy line represents that the compound has either *E* or *Z* configuration.]

In General Formula (IV) or General Formula (V), the number of carbon atoms in G is not limited as long as it is alkyl, and the alkyl may be linear, branched, or cyclic. Examples of the alkyl include $C_1$-$C_{10}$ alkyls (such as methyl, ethyl, propyl, and adamantyl) and $C_1$-$C_4$ alkyls (such as methyl, ethyl, and propyl).

In a preferred embodiment, the anthracycline drug equivalent of General Formula (IV) or General Formula (V) may be a compound of any of the following formulae.

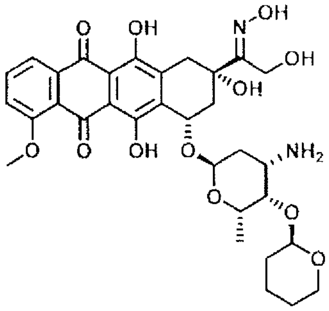
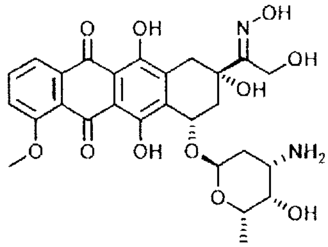
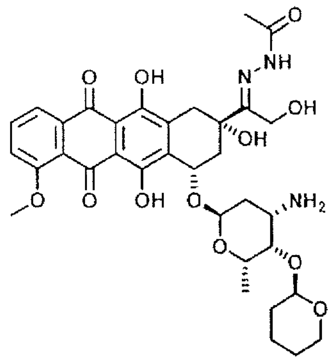

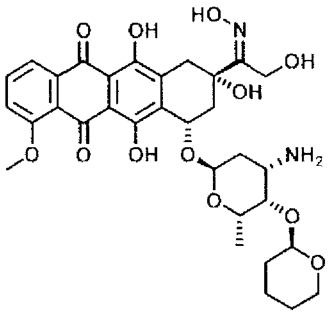
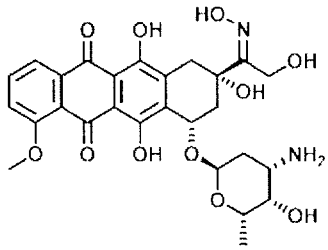
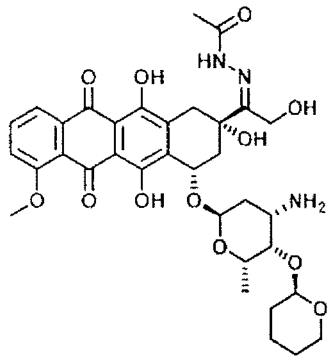

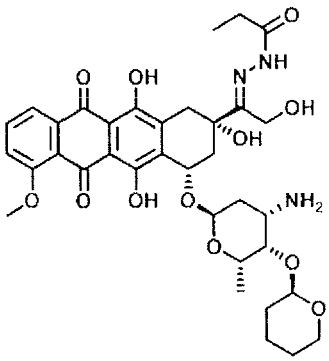
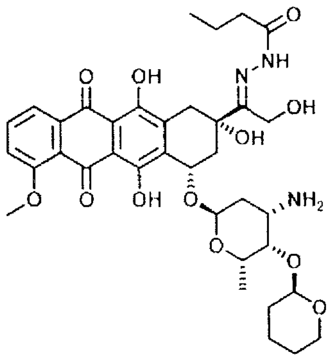
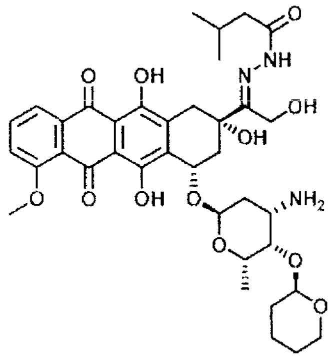

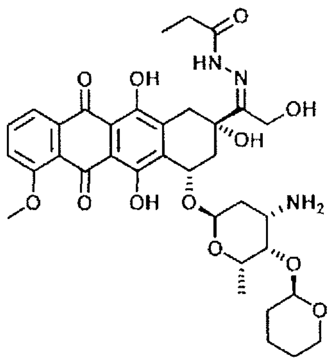
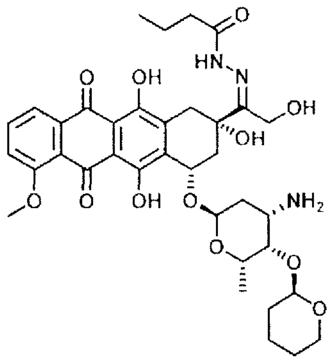
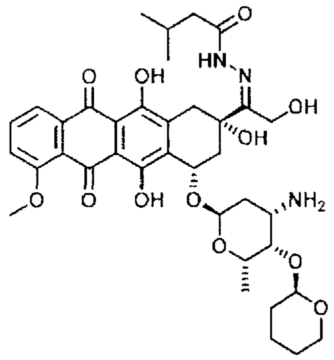

Examples of the protonic acid in the method of evaluating the purity according to the present invention include inorganic acids such as hydrochloric acid, sulfuric acid, phosphoric acid, and hydrobromic acid; carboxylic acids such as formic acid, acetic acid, propionic acid, butyric acid, valeric acid, caproic acid, and benzoic acid; sulfonic acids such as methanesulfonic acid and *p*-toluenesulfonic acid; and ascorbic acid. From the viewpoint of the acidity, carboxylic acids are preferred, and acetic acid is more preferred. The protonic acid used may be a single compound, or may be a combination of two or more kinds of compounds.

The amount of the nitrogen-containing nucleophile used is preferably 0.1 to 100 times the weight, more preferably 0.5 to 50 times the weight of the complex from the viewpoint of obtaining a sufficient reaction conversion rate and suppressing side reactions. The amount of the protonic acid used is preferably 0.1 to 100 times the weight, more preferably 0.5 to 50 times the weight of the complex from the viewpoint of obtaining a sufficient reaction conversion rate and suppressing side reactions.

Examples of the polar solvent in the method of evaluating the purity of the drug according to the present invention include ether solvents such as tetrahydrofuran and dimethoxyethane; nitrile solvents such as acetonitrile and propionitrile; amide solvents such as *N,N*-dimethylformamide, *N,N*-dimethylacetamide, and *N*-methylpyrrolidone; sulfoxide solvents such as dimethylsulfoxide; urea solvents such as 1,3-dimethyl-2-imidarolidinone; alcoholic solvents such as methanol, ethanol, and 2-propanol; and mixed solvents thereof. From the viewpoint of dissolving the complex, alcoholic solvents such as methanol, ethanol, and 2-propanol are preferred, and methanol is more preferred. The amount of the solvent used is preferably 3 to 100 times the weight, more preferably 5 to 20 times the weight of the complex from the viewpoint of suppressing side reactions.

In the method of evaluating the purity according to the present invention, a preferred mode of the nitrogen-containing nucleophile, a preferred mode of the protonic acid, and a preferred mode of the polar solvent may be arbitrarily combined. Examples of the combination include hydroxylamine, a carboxylic acid, and an alcoholic solvent; an *O*-alkylhydroxylamine, a carboxylic acid, and an alcoholic solvent; and a carboxylic acid hydrazide, a carboxylic acid, and an alcoholic solvent. The combination is preferably hydroxylamine, acetic acid, and methanol; acetohydrazide, acetic acid, and methanol; propanohydrazide, acetic acid, and methanol; butyrohydrazide, acetic acid, and methanol; or 3-methylbutanohydrazide, acetic acid, and methanol.

The nitrogen-containing nucleophile, the protonic acid, and the polar solvent may be labeled with a radioisotope, or may be deuterated substances.

The reaction temperature in the reaction step in the method of evaluating the purity according to the present invention is preferably -10°C to 30°C, more preferably -5°C to 5°C from the viewpoint of suppressing side reactions. The reaction time may be appropriately selected depending on conditions such as the reaction temperature. From the viewpoint of suppressing side reactions, the reaction time is preferably 1 to 40 hours, more preferably 3 to 30 hours.

2. Complex (I) or Pharmaceutically Acceptable Salt Thereof

When the complex of General Formula (I):

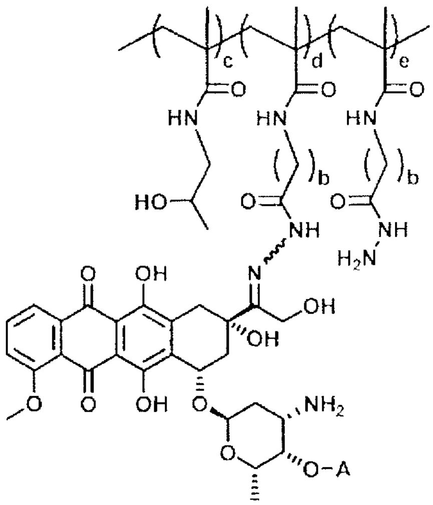

(I)

[In the formula, A is a hydrogen atom or (*R*)-tetrahydro-2*H*-pyran-2-yl; b, c, d, and e each independently are a positive integer; and the bond indicated by a wavy line represents that the compound has either *E* or *Z* configuration.]

or the pharmaceutically acceptable salt thereof according to the present invention is evaluated by the purity evaluation method described above, the purity of the drug contained in Complex (I) or the pharmaceutically acceptable salt thereof is not less than 95.0%.

From the viewpoint of the efficiency of transport by a transporter, A is preferably (*R*)-tetrahydro-2*H*-pyran-2-yl.

Although b, c, d, and e are not limited as long as they are positive integers, b is preferably an integer of 1 to 10 (for example, 5); c is preferably an integer of 30 to 500, especially preferably an integer of 50 to 500; d is preferably

an integer of 1 to 50; and e is preferably an integer of 1 to 50.

In a preferred embodiment, the complex of General Formula (1) or the pharmaceutically acceptable salt thereof may be of the following General Formula (I'):

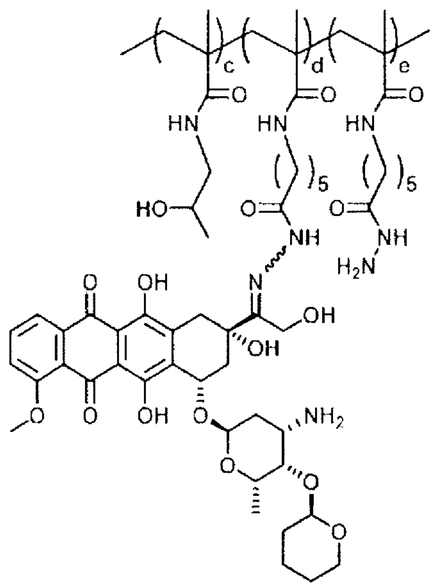

(I')

[In the formula, c, d, and e each independently are a positive integer; and the bond indicated by a wavy line represents that the compound has either *E* or *Z* configuration.]

Although c, d, and e in General Formula (I') are not limited as long as they are positive integers, c is preferably an integer of 30 to 500, especially preferably an integer of 50 to 500; d is preferably an integer of 1 to 50; and e is preferably an integer of 1 to 50.

Examples of the pharmaceutically acceptable salt of the complex of General Formula (I) include inorganic acid salts such as hydrochloride, sulfate, phosphate, and hydrobromide; and organic acid salts such as oxalate, malonatc, citrate, fumarate, lactate, malate, succinate, tartrate, acetate, trifluoroacetate, maleate, gluconate, benzoate, salicylate, xinafoate, pamoate, ascorbate, adipate, methanesulfonate, *p*-toluenesulfonate, and cinnamate. These salts may be in the form of a hydrate, solvate, or crystal polymorphism.

Complex (1) or the pharmaceutically acceptable salt thereof may be labeled with a radioisotope, or may be a deuterated substance.

Examples of Complex (I) or the pharmaceutically acceptable salt thereof include its hydrates, solvates, crystal polymorphisms, and labeled bodies, and mixtures thereof.

From the viewpoint of satisfying the THP purity standard described in the Japanese Pharmacopoeia, the bound-drug purity of Complex (I) or the pharmaceutically acceptable salt thereof according to the present invention is not less than 95.0%, preferably not less than 96.0%, more preferably not less than 97.0% as evaluated by the same method as described in the method of evaluating the purity of the drug contained in the complex. Also taking into account the purity standard for DOX hydrochloride described in the Japanese Pharmacopoeia, the bound-drug purity is preferably not less than 98.0%. The closer the bound-drug purity to 100%, the more preferred. By the later-described production method of the present invention, a product having a bound-drug purity of up to 99.5% has been obtained.

In general, pharmaceuticals are subjected to purity evaluation for the purpose of quality confirmation. For example, according to the definition on the purity of THP by the Japanese Pharmacopoeia, THP should show a titer of not less than 95.0% depending on the quantification method, and a high-purity pharmaceutical has been thought to be desirable from the viewpoint of the quality. On the other hand, when the complex is used as a pharmaceutical, the anthracycline drug released from the complex produces the pharmacological effect. Therefore, the purity of the drug contained in the complex, that is, the bound-drug purity, corresponds to the purity of an ordinary pharmaceutical, so that evaluation of the bound-drug purity is very important for the quality control of the pharmaceutical. Thus, the higher the bound-drug purity, the better from the viewpoint of the quality control of the pharmaceutical. In cases where THP is used as the anthracycline drug, at least the THP purity standard described in the Japanese Pharmacopoeia needs to be satisfied. Also taking into account cases where DOX hydrochloride is used as the anthracycline drug, the purity standard for DOX hydrochloride described in the Japanese Pharmacopoeia is preferably satisfied.

From the viewpoint of drug transport, the amount of the carried bound drug in Complex (I) or the pharmaceutically acceptable salt thereof is preferably 3 to 20 wt%, more preferably 4 to 19 wt%, still more preferably 7 to 17 wt%. On the

other hand, from the viewpoint of storage stability, the amount of the carried bound drug in Complex (I) or the pharmaceutically acceptable salt thereof is preferably 4 to 20 wt%, more preferably 10 to 19 wt%, still more preferably 15 to 19 wt%.

In the present description, "the amount of the carried bound drug" means an estimated amount of the carried drug contained in Complex (I) or the pharmaceutically acceptable salt thereof, and can be calculated by quantitative analysis of the measurement target by the following method according to the method of evaluating the purity of a drug contained in Complex (I) or the pharmaceutically acceptable salt. Based on a chromatogram of HPLC obtained by the following method, the number of moles of the measurement target peak is calculated by quantitative analysis using the HPLC area value of the measurement target peak and a calibration curve prepared from a corresponding measurement target authentic sample, and the weight of the corresponding active pharmaceutical ingredient is calculated from the obtained number of moles, followed by calculating the amount of the carried bound drug according to the following calculation equation.

Amount of carried bound drug (wt%) = weight of active pharmaceutical ingredient (mg) / weight of complex (mg) $\times$ 100

The measurement target peak means the peak of the equivalent of the anthracycline drug based on the molecular weight. In cases where separated peaks of isomers appear under the above analysis conditions, the sum of the area percentages of the peaks of the isomers is used as the HPLC area value of the measurement target peak.

(1) Sample Preparation

In a 2-mL measuring flask, an accurately weighed complex (20$\pm$0.4 mg) is placed, and methanol (1 mL) is added thereto to dissolve the complex, followed by adding 50% by weight aqueous hydroxylamine solution (320 μL) and acetic acid (270 μL) to the resulting solution, and then accurately adding methanol thereto to a final volume of 2 mL. The obtained solution is stirred at 0°C for 25 hours to provide a measurement sample.

(2) HPLC

The measurement sample prepared as described above is analyzed using HPLC under the following measurement conditions.

Detector: photodiode array detector (measurement wavelength: 488 nm)
Column: Scherzo SM-C18 (manufactured by Imtakt Corporation)
Column size: 150$\times$4.6 mm (3 μm)
Column temperature: 30°C
Mobile phase:
Solution A, 5 mmol/L ammonium formate reagent solution / methanol mixed solution (95:5)
Solution B, methanol / water / formic acid mixed solution (90:10:0.1)
Developing condition:

A/B = 75/25 (minute 0 to 3)
A/B = 75/25 to 37/63 (minute 3 to 4; linear gradient)
A/B = 37/63 (minute 4 to 24)
A/B = 37/63 to 20/80 (minute 24 to 25; linear gradient)
A/B = 20/80 (minute 25 to 35)
A/B = 20/80 to 0/100 (minute 35 to 39; linear gradient)
A/B = 0/100 (minute 39 to 54)
A/B = 0/100 to 75/25 (minute 54 to 54.1; linear gradient)
A/B = 75/25 (minute 54.1 to 59.5)

Flow rate: 1.0 mL/minute
Injection volume: 10 μL
Sample cooler temperature: 4°C

From the viewpoint of safety, the weight average molecular weight of Complex (I) or the pharmaceutically acceptable salt thereof is preferably 25,000 to 85,000, more preferably 27,000 to 65,000, still more preferably 30,000 to 50,000.

In the present description, "weight average molecular weight" means a weight average molecular weight calculated according to the following method.

(1) Sample Preparation

Methanol (500 μL) is added to a complex (5 mg), to provide a measurement sample.

(2) Weight Average Molecular Weight

Each measurement sample prepared as described above is subjected to analysis using HPLC and MALS under the following conditions, and the weight average molecular weight (Mw) is calculated using analysis software [ASTRA Ver. 7.3.2.17 64-bit (Wyatt technology)] (refractive index increment: dn/dc = 0.175).

HPLC: LC-40 (manufactured by Shimadzu Corporation)
Detectors: a photodiode array detector (measurement wavelength: 488 nm) and a differential refractometer
Columns: TSKgel α-M (manufactured by Tosoh Corporation) and TSKgel α-2500 (manufactured by Tosoh Corporation), sequentially connected
Column sizes:

TSKgel α-M (300 × 7.8 mm (7 μm))
TSKgel α-2500 (300 × 7.8 mm (7 μm))
Column temperature: 30°C
Mobile phase: methanol : 0.3 mol/L sodium acetate reagent solution (pH 6.5) = 80:20
Flow rate: 0.8 mL/minute
Injection volume: 20 μL
Sample cooler temperature: 4°C
Injector washing liquid: methanol : water = 80:20
MALS: DAWN8 (Wyatt technology)
Light Scattering Instrument:
Calibration constant: 5.2929/100,000 [1/Vcm]
RI Instrument:
Refraction constant: 1.338

In Complex (1) and the pharmaceutically acceptable salt thereof, a preferred mode of the bound-drug purity, a preferred mode of the amount of the carried bound drug, and a preferred mode of the weight average molecular weight may be arbitrarily combined. Examples of the combination include Complex (1) or a pharmaceutically acceptable salt thereof in which the bound-drug purity is not less than 95.0%; the amount of the carried bound drug is 3 to 20 wt%; and the weight average molecular weight is 25,000 to 85,000.

In general, a pharmaceutical needs to be evaluated for its stability in order to specify the storage conditions and the shelf life. Representative examples of the stability test for a pharmaceutical include a stress test, a long-term storage test, and an acceleration test. For example, a stress test of an active pharmaceutical ingredient is utilized for identification of degradation products that may be produced from the active pharmaceutical ingredient, confirmation of compatibility of analysis methods to such degradation products, prediction of the stability of the active pharmaceutical ingredient, and the like. A stress test at 60°C under airtight conditions for 4 weeks corresponds to three years of storage at 25°C under airtight conditions, and is utilized for prediction of the stability at an initial stage of the development (Sumie Yoshioka, Stability of Medicines, Nankodo Co., Ltd., 1995, p. 142).

Complex (1) or a pharmaceutically acceptable salt thereof having a bound-drug purity of not less than 95.0% as evaluated by the above purity evaluation method can be obtained by the following production method.

3. Method of Producing Complex (I) or Pharmaceutically Acceptable Salt Thereof

The method of producing a complex of General Formula (I):

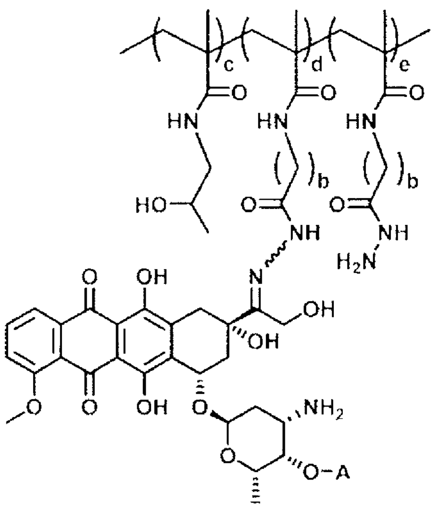

(I)

or a pharmaceutically acceptable salt thereof according to the present invention comprises a reaction step of reacting an anthracycline drug of General Formula (II):

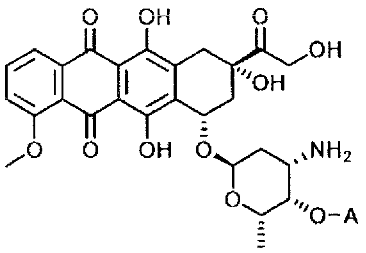

(I I)

with an *N*-(2-hydroxypropyl)methacrylamide polymer of General Formula (III):

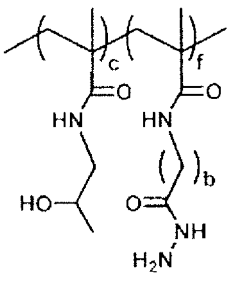

(I I I)

in the presence of a protonic acid in a polar solvent at not more than 10°C, to obtain the complex of General Formula (I) or the pharmaceutically acceptable salt thereof
[wherein in Formula (I) and Formula (II), A is a hydrogen atom or (R)-tetrahydro-2*H*-pyran-2-yl; and, in Formula (1) and Formula (III), b, c, d, e, and f each independently are a positive integer, and the bond indicated by a wavy line represents that the complex has either *E* or *Z* configuration].

From the viewpoint of the efficiency of transport by a transporter, A is preferably (*R*)-tetrahydro-2*H*-pyran-2-yl.

Although b, c, d, and e are not limited as long as they are positive integers, b is preferably an integer of 1 to 10 (for example, 5); c is preferably an integer of 30 to 500, especially preferably an integer of 50 to 500; d is preferably an integer of 1 to 50; and e is preferably an integer of 1 to 50. f is the sum of d and e.

The method of producing Complex (I) or a pharmaceutically acceptable salt thereof according to the present invention can be used especially as a method of producing Complex (I) or a pharmaceutically acceptable salt thereof having a bound-drug purity of not less than 95.0% as evaluated by the purity evaluation method described above.

In the purification of Complex (I) or a pharmaceutically acceptable salt thereof, low molecular weight compounds such as an unreacted anthracycline drug and an anthracycline drug produced by cleavage of the hydrazone bond can

be removed by a purification operation such as column chromatography, thin-layer chromatography, recrystallization, or reprecipitation. However, unlike in the cases of ordinary low molecular weight organic compounds, the bound-drug purity cannot be increased. In other words, even in cases where a partial structure other than the hydrazone bond of the anthracycline drug on the complex is degraded, separation of the degradation product of the drug from the complex does not occur unless the hydrazone bond is cleaved, so that the bound-drug purity does not increase. Thus, the reaction step in the production of the complex is very important from the viewpoint of the quality control, and the bound-drug purity is dependent on how degradation of the anthracycline drug during the reaction step is suppressed.

In the method of producing Complex (1) or a pharmaceutically acceptable salt thereof, the amount of the *N*-(2-hydroxypropyl)methacrylamide polymer used in the reaction step may be appropriately selected in accordance with the desired amount of the carried bound drug. From the viewpoint of the drug transport efficiency, the amount of the *N*-(2-hydroxypropyl)methacrylamide polymer is preferably 1 to 30 times the weight, more preferably 3 to 25 times the weight, still more preferably 3 to 15 times the weight of the anthracycline drug.

In the method of producing Complex (1) or a pharmaceutically acceptable salt thereof, examples of the protonic acid used in the reaction step include inorganic acids such as hydrochloric acid, sulfuric acid, phosphoric acid, and hydrobromic acid; and organic acids such as oxalic acid, malonic acid, citric acid, fumaric acid, lactic acid, malic acid, succinic acid, tartaric acid, acetic acid, trifluoroacetic acid, maleic acid, gluconic acid, benzoic acid, salicylic acid, xinafoic acid, pamoic acid, ascorbic acid, adipic acid, methanesulfonic acid, *p*-toluenesulfonic acid, and cinnamic acid. From the viewpoint of the acidity, organic acids are preferred, and acetic acid is more preferred. The protonic acid used may be a single compound, or may be a combination of two or more kinds of compounds.

In the method of producing Complex (I) or a pharmaceutically acceptable salt thereof, the amount of the protonic acid used in the reaction step is preferably 10 to 300 molar equivalents, more preferably 20 to 200 molar equivalents relative to the amount of the anthracycline drug from the viewpoint of obtaining a sufficient reaction conversion rate. The "molar equivalent" herein means the number of moles of the protonic acid used per 1 mole of the anthracycline drug.

Examples of the polar solvent used in the reaction step in the method of producing Complex (1) or a pharmaceutically acceptable salt thereof include ether solvents such as tetrahydrofuran and dimethoxyethane; nitrile solvents such as acetonitrile and propionitrile; amide solvents such as *N*,*N*-dimethylformamide, *N,N*-dimethylacetamide, and *N*-methylpyrrolidone; sulfoxide solvents such as dimethylsulfoxide; urea solvents such as 1,3-dimethyl-2-imidazolidinone; alcoholic solvents such as methanol, ethanol, and 2-propanol; and mixed solvents thereof. From the viewpoint of dissolving the complex, alcoholic solvents such as methanol, ethanol, and 2-propanol are preferred, and methanol is more preferred. The amount of the solvent used is preferably 10 to 200 times the weight, more preferably 20 to 70 times the weight of the anthracycline drug from the viewpoint of simply carrying out stirring and increasing the production efficiency per unit volume.

In the method of producing Complex (I) or a pharmaceutically acceptable salt thereof, from the viewpoint of suppressing side reactions, the reaction temperature in the reaction step is not more than 10°C. From the viewpoint of increasing the production efficiency per unit time, the reaction temperature is preferably -30°C to 10°C, especially preferably -30°C to 5°C. The reaction time may be appropriately selected depending on conditions such as the reaction temperature. From the viewpoint of increasing the production efficiency per unit time, the reaction time is preferably 1 to 300 hours, more preferably 10 to 150 hours.

In the method of producing Complex (I) or a pharmaceutically acceptable salt thereof, a preferred mode of the polar solvent, a preferred mode of the protonic acid, and a preferred mode of the reaction temperature may be arbitrarily combined. Examples of the combination include an alcoholic solvent, an organic acid, and a reaction temperature of not more than 10°C. The combination of methanol, acetic acid, and a reaction temperature of -30°C to 10°C, especially preferably -30°C to 5°C, is preferred.

In the method of producing Complex (I) or a pharmaceutically acceptable salt thereof, the order of addition of the polar solvent, the anthracycline drug of General Formula (II), the *N*-(2-hydroxypropyl)methacrylamide polymer of General Formula (III), and the protonic acid used in the reaction step is not limited. From the viewpoint of obtaining a sufficient reaction conversion rate and suppressing side reactions, it is preferred to add the *N*-(2-hydroxypropyl)methacrylamide polymer of General Formula (III) to the polar solvent to dissolve the polymer in the solvent, followed by adding the the anthracycline drug of General Formula (II) to the resulting solution, and then adding the protonic acid thereto.

As the anthracycline drug of General Formula (II), a commercially available product may be used as it is. For example, THP (wherein A is (*R*)-tetrahydro-2*H*-pyran-2-yl) can be purchased from MicroBiopharm Japan Co., Ltd. (quantitative method: 95.0% or higher). DOX hydrochloride (wherein A is a hydrogen atom) can be purchased from MedKoo Biosciences, Inc. (HPLC purity: 99.0% or higher). Further, as the *N*-(2-hydroxypropyl)methacrylamide polymer of General Formula (III), a commercially available product may be used as it is. For example, it can be purchased from the Institute of Macromolecular Chemistry, Czech Academy of Sciences, or from Chemicalsoft Co., Ltd.

In the *N*-(2-hydroxypropyl)methacrylamide polymer of General Formula (III), the positions of the hydrazide groups are not limited, and the hydrazide groups may be positioned either regularly or randomly. For example, two or more monomer units containing a hydrazide group may be continuously linked. The *N*-(2-hydroxypropyl)methacrylamide

polymer is thought to have, as an end structure, a saturated or unsaturated end structure (disproportionated end formed by hydrogen abstraction), a dimethylnitrile end structure (radical initiator end derived from azobisisobutyronitrile), or the like.

The *N*-(2-hydroxypropyl)methacrylamide polymer of General Formula (III) that can be purchased from the Institute of Macromolecular Chemistry, Czech Academy of Sciences, or from Chemicalsoft Co., Ltd. has a weight average molecular weight of 20,000 to 35,000, and the content of hydrazide groups that are the copolymer component is 5.0 to 7.0 mol%.

Complex (1) or the pharmaceutically acceptable salt thereof can be purified by a method such as column chromatography, thin-layer chromatography, recrystallization, or reprecipitation. Those skilled in the art can select or combine, from these methods, a method(s) suitable for a specific target compound, and can easily optimize the purification method. In the production of Complex (I) or the pharmaceutically acceptable salt thereof by the production method according to the present invention, only a small amount of by-products are generated, so that it can be obtained by a simple isolation/purification operation. Taking commercial production into account, recrystallization or reprecipitation using a mixed solvent of ethyl acetate and methanol is preferred.

The method of producing Complex (I) or a pharmaceutically acceptable salt thereof may comprise a precipitation step and/or a drying step after the reaction step.

Examples of the solvent used in the precipitation step include ether solvents such as tetrahydrofuran and dimethoxyethane; nitrile solvents such as acetonitrile and propionitrile; ester solvents such as ethyl acetate and isopropyl acetate; alcoholic solvents such as methanol, ethanol, and 2-propanol; and mixed solvents thereof. From the viewpoint of achieving high recovery of the complex and enabling simple solvent removal by evaporation, a mixed solvent of an alcoholic solvent and an ester solvent is preferred, and a mixed solvent of methanol and ethyl acetate is more preferred.

The amount of the mixed solvent used is preferably 50 to 1000 times the weight, more preferably 100 to 350 times the weight of the anthracycline drug of General Formula (II), from the viewpoint of simply carrying out stirring and increasing the production efficiency per unit volume. The ratio of the ester solvent in the mixed solvent is preferably 1 to 10 times the weight, more preferably 2 to 6 times the weight of the alcoholic solvent, from the viewpoint of achieving high recovery of the complex and enabling removal of the unreacted anthracycline drug.

The stirring temperature in the precipitation step is preferably -20°C to 50°C, more preferably -10°C to 40°C, from the viewpoint of suppressing the degradation of the complex and enabling removal of the unreacted anthracycline drug. The stirring time in the precipitation step is preferably 0.1 to 100 hours, more preferably 0.25 to 50 hours, from the viewpoint of suppressing the degradation of the complex and enabling removal of the unreacted anthracycline drug.

The drying temperature in the drying step is preferably -10°C to 50°C, more preferably 0°C to 40°C, from the viewpoint of suppressing the degradation of the complex and quickly removing the residual solvent. The drying method is not limited, and, from the viewpoint of quickly removing the residual solvent, drying under reduced pressure is preferred. The degree of reduction of the pressure during the drying under reduced pressure is preferably not more than 500 Pa from the viewpoint of quickly removing the residual solvent.

## EXAMPLES

The present invention will now be described below concretely by way of Examples. However, the present invention is not limited thereto. First, the method of evaluating the purity is described.

### (1) Sample Preparation

To a solution of the solid (10 mg) obtained by each of the later-described Examples 2 to 15, Comparative Example 1, and Comparative Example 2 in methanol (160 μL), 50% by weight aqueous hydroxylamine solution (32 μL) and acetic acid (27 μL) were added at 0°C, and the resulting mixture was stirred at 0°C for 25 hours. Methanol (450 μL) was added to the obtained reaction mixture (50 μL), to provide a measurement sample.

### (2) HPLC

Each measurement sample prepared as described above was subjected to analysis using HPLC under the following measurement conditions.

HPLC: LC-20AD (manufactured by Shimadzu Corporation)
Detector: photodiode array detector (measurement wavelength: 488 nm)
Column: Scherzo SM-C18 (manufactured by Imtakt Corporation)
Column size: 150×4.6 mm (3 μm)
Column temperature: 30°C

Mobile phase:

Solution A, 5 mmol/L ammonium formate reagent solution / methanol mixed solution (95:5)
Solution B, methanol / water / formic acid mixed solution (90:10:0.1)
Developing condition:

A/B = 75/25 (minute 0 to 3)
A/B = 75/25 to 37/63 (minute 3 to 4; linear gradient)
A/B = 37/63 (minute 4 to 24)
A/B = 37/63 to 20/80 (minute 24 to 25; linear gradient)
A/B = 20/80 (minute 25 to 35)
A/B = 20/80 to 0/100 (minute 35 to 39; linear gradient)
A/B = 0/100 (minute 39 to 54)
A/B = 0/100 to 75/25 (minute 54 to 54.1; linear gradient)
A/B = 75/25 (minute 54.1 to 59.5)

Flow rate: 1.0 mL/minute
Injection volume: 20 μL
Sample cooler temperature: 4°C
Injector washing liquid: acetonitrile/water mixed liquid (60:40)

(3) Bound-Drug Purity

Based on the HPLC chromatogram obtained by the measurement, the area percentage of the measurement target peak as the bound-drug purity was calculated taking the total peaks excluding the blank peak as 100%. The blank peak means the peak detected when methanol is measured under the following HPLC conditions. The measurement target peak means the peak of the anthracycline drug equivalent based on the molecular weight. In cases where separated peaks of isomers appeared under the above analysis conditions, the sum of the area percentages of the peaks of the isomers was used as the area percentage of the measurement target peak.

In the following, the method of evaluating the amount of the carried bound drug is described.

(1) Sample Preparation

In a 2-mL measuring flask, an accurately weighed solid (20±0.4 mg) obtained in each of the later-described Examples 2 to 15, Comparative Example 1, and Comparative Example 2 was placed, and methanol (1 mL) was added thereto to dissolve the solid, followed by adding 50% by weight aqueous hydroxylamine solution (320 μL) and acetic acid (270 μL) to the resulting solution, and then accurately adding methanol thereto to a final volume of 2 mL. The obtained solution was stirred at 0°C for 25 hours to provide a measurement sample.

(2) HPLC

The measurement sample prepared as described above was subjected to analysis using HPLC under the following measurement conditions.

Detector: photodiode array detector (measurement wavelength: 488 nm)
Column: Scherzo SM-C18 (manufactured by Imtakt Corporation)
Column size: 150×4.6 mm (3 μm)
Column temperature: 30°C
Mobile phase:

Solution A, 5 mmol/L ammonium formate reagent solution / methanol mixed solution (95:5)
Solution B, methanol / water / formic acid mixed solution (90:10:0.1)

Developing condition:

A/B = 75/25 (minute 0 to 3)
A/B = 75/25 to 37/63 (minute 3 to 4; linear gradient)
A/B = 37/63 (minute 4 to 24)

A/B = 37/63 to 20/80 (minute 24 to 25; linear gradient)
A/B = 20/80 (minute 25 to 35)
A/B = 20/80 to 0/100 (minute 35 to 39; linear gradient)
A/B = 0/100 (minute 39 to 54)
A/B = 0/100 to 75/25 (minute 54 to 54.1; linear gradient)
A/B = 75/25 (minute 54.1 to 59.5)

Flow rate: 1.0 mL/minute
Injection volume: 10 μL
Sample cooler temperature: 4°C

(3) Amount of Carried bound Drug

Based on the chromatogram of HPLC obtained by the above measurement, the number of moles of the measurement target peak was calculated by quantitative analysis using the HPLC area value of the measurement target peak and a calibration curve prepared from a corresponding measurement target authentic sample, and the weight of the corresponding active pharmaceutical ingredient was calculated from the obtained number of moles, followed by calculating the amount of the carried bound drug according to the following calculation equation.

$$\text{Amount of carried bound drug (wt\%)} = \text{weight of active pharmaceutical ingredient (mg)} / \text{weight of complex (mg)} \times 100$$

The measurement target peak means the peak of the anthracycline drug equivalent based on the molecular weight. In cases where separated peaks of isomers appeared under the above analysis conditions, the sum of the area percentages of the peaks of the isomers was used as the HPLC area value of the measurement target peak.
The measurement target authentic sample can be synthesized by the following method.

Synthesis Example 1

Synthesis of THP Equivalent Using Hydroxylamine (Measurement Target Authentic Sample):

To a suspension of THP (625 mg, manufactured by MicroBiopharm Japan Co., Ltd.) in methanol (8.0 mL), 50% by weight aqueous hydroxylamine solution (530 μL) and acetic acid (460 μL) were added at 0°C, and the resulting mixture was stirred at 0°C for 25 hours. The obtained reaction mixture was purified by silica gel column chromatography, and then acetonitrile (40 mL) and diisopropyl ether (100 mL) were added thereto, followed by stirring the resulting mixture at 25°C for 2 hours. After collecting the precipitated solid by filtration, the solid was dried at 25°C under reduced pressure, to obtain a THP equivalent (yield, 506 mg; percent yield, 79%).

Synthesis Example 2

Synthesis of DOX Equivalent Using Hydroxylamine (Measurement Target Authentic Sample):

To a suspension of DOX hydrochloride (500 mg, manufactured by MedKoo Biosciences, Inc.) in methanol (9.1 mL), 50% by weight aqueous hydroxylamine solution (610 μL) and acetic acid (530 μL) were added at 0°C, and the resulting mixture was stirred at 0°C for 25 hours. To the obtained reaction mixture, methanol (1.0 mL) and acetonitrile (20 mL) were added, and the resulting mixture was stirred at 25°C for 2 hours. After collecting the precipitated solid by filtration, the solid was dried at 25°C under reduced pressure, to obtain a DOX equivalent (yield, 464 mg; percent yield, 90%).
In the following, the method of evaluating the weight average molecular weight is described.

(1) Sample Preparation

To the solid (5 mg) obtained in each of the later-described Examples 2 to 15, Comparative Example 1, and Comparative Example 2, methanol (500 μL) was added to provide a measurement sample.

(2) Weight Average Molecular Weight

Each measurement sample prepared as described above was subjected to analysis using IIPLC and MALS under the following conditions, and its weight average molecular weight (Mw) was calculated using analysis software [ASTRA Ver. 7.3.2.17 64-bit (Wyatt technology)] (refractive index increment: dn/dc = 0.175).

HPLC: LC-40 (manufactured by Shimadzu Corporation)
Detectors: a photodiode array detector (measurement wavelength: 488 nm) and a differential refractometer
Columns: TSKgel α-M (manufactured by Tosoh Corporation) and TSKgel α-2500 (manufactured by Tosoh Corporation), sequentially connected
Column sizes:

TSKgcl α-M (300 × 7.8 mm (7 μm))
TSKgel α-2500 (300 × 7.8 mm (7 μm))

Column temperature: 30°C
Mobile phase: methanol : 0.3 mol/L sodium acetate reagent solution (pH 6.5) = 80:20
Flow rate: 0.8 mL/minute
Injection volume: 20 μL
Sample cooler temperature: 4°C
Injector washing liquid: methanol : water = 80:20
MALS: DAWN8 (Wyatt technology)
Light Scattering Instrument:
Calibration constant: 5.2929/100,000 [1/Vcm]
RI Instrument:
Refraction constant: 1.338

Example 1

Evaluation of Bound-Drug Purity:

In the following, Test Examples 1 to 11 show the results of studies on the method of evaluation of the bound-drug purity.

Test Example 1

Hydrolysis of P-TIIP (Comparative Example 1) Using Method Described in Non-Patent Document 2:

To the P-THP (20 mg) obtained by Comparative Example 1, 1 mol/L hydrochloric acid (2.0 mL) was added, and the resulting mixture was stirred at 85°C for 20 minutes. The obtained reaction mixture was used as a measurement sample. The obtained measurement sample was subjected to HPLC measurement by the method described above. As a result, the purity of THP was found to be 1.1%.

Test Example 2

Hydrolysis of P-THP (Comparative Example 1) According to Method Described in Non-Patent Document 2 (Reaction Temperature, 0°C; Hydrochloric Acid):

To the P-THP (20 mg) obtained by Comparative Example 1, 1 mol/L hydrochloric acid (2.0 mL, 0°C) that had been cooled was added, and the resulting mixture was stirred at 0°C for 1 hour. The obtained reaction mixture was used as a measurement sample. The obtained measurement sample was subjected to HPLC measurement by the method described above. As a result, the purity of THP was found to be 0.2%.

Test Example 3

Hydrolysis of P-THP (Comparative Example 1) According to Method Described in Non-Patent Document 2 (Reaction Temperature, 0°C; Acetic Acid):

To the P-THP (20 mg) obtained by Comparative Example 1, 1 mol/L aqueous acetic acid solution (2.0 mL, 0°C) that had been cooled was added, and the resulting mixture was stirred at 0°C for 2 hours. The obtained reaction mixture was used as a measurement sample. The obtained measurement sample was subjected to HPLC measurement by the method described above. As a result, the purity of THP was found to be 83.3%.

Test Example 4

Conversion of THP Using Hydroxylamine and Acetic Acid:

To a suspension of THP (1.0 mg, manufactured by MicroBiopharm Japan Co., Ltd.) in methanol (160 μL), 50% by weight aqueous hydroxylamine solution (32 μL) and acetic acid (27 μL) were added at 0°C, and the resulting mixture was stirred at 0°C for 25 hours. Methanol (450 μL) was added to the obtained reaction mixture (50 μL), to provide a measurement sample. The obtained measurement sample was subjected to HPLC measurement by the method described above. As a result, the purity of the THP equivalent was found to be 98.9%.

Test Example 5

Evaluation of Purity of THP:

To a suspension of THP (5.0 mg, manufactured by MicroBiopharm Japan Co., Ltd.) in methanol (6.0 mL), *N*,*N*-dimethylformamide (4.0 mL) was added to provide a measurement sample. The obtained measurement sample was subjected to HPLC measurement by the method described above. As a result, the purity of THP was found to be 99.0%.

The HPLC analysis results of Test Examples 1 to 5 are shown in Table 1.

[Table 1]

| | Complex | Additive | Reaction temperature (°C) | HPLC area percentage (%) | | | | |
|---|---|---|---|---|---|---|---|---|
| | | | | DOX equivalent | DOX | THP equivalent | THP | P-THP |
| Test Example 1 | P-THP (Comparative Example 1) | Hydrochloric acid | 85 | - | 3.1 | - | 1.1 | 3.0 |
| Test Example 2 | P-THP (Comparative Example 1) | Hydrochloric acid | 0 | - | 93.5 | - | 0.2 | 4.8 |
| Test Example 3 | P-THP (Comparative Example 1) | Acetic acid | 0 | - | 6.9 | - | 83.3 | 9.3 |
| Test Example 4 | THP was used instead | Hydroxylamine and acetic acid | 0 | 0.1 | <0.1 | 98.9 | 0.1 | - |
| Test Example 5 | THP was used instead | - | - | - | 0.1 | - | 99.0 | - |

As shown in Table 1, the method described in Non-Patent Document 2 was found to cause release of THP from the complex and degradation of THP into substances having unknown structures (Test Example 1). Further, under the hydrolysis conditions using hydrochloric acid at a reaction temperature of 0°C according to the method described in Non-Patent Document 2, conversion of THP to DOX occurred upon the release of THP. Thus, calculation of the bound-drug purity was difficult even by the use of this method (Test Example 2). Further, under the hydrolysis conditions using acetic acid at a reaction temperature of 0°C according to the method described in Non-Patent Document 2, the rate of conversion to THP was high, but the conversion of THP to DOX could not be completely suppressed, so that calculation of the bound-drug purity was difficult even by the use of this method (Test Example 3). In contrast, when THP was used instead of the complex, and hydroxylamine and acetic acid were used as additives, the conversion from THP to DOX hardly proceeded, and it was found that the THP equivalent could be obtained with a good purity based on comparison with the result of evaluation of the purity of the THP used (Test Examples 4 and 5). From these results, it was thought that evaluation of the bound-drug purity would be possible if a similar reaction proceeds when the complex is used under the reaction conditions of Test Example 4.

Test Example 6

Evaluation of Bound-Drug Purity of P-THP (Comparative Example 1) Using Hydroxylamine, Acetic Acid, and Methanol:

To a solution of the P-THP (10 mg) obtained by Comparative Example 1 in methanol (160 μL), 50% by weight aqueous hydroxylamine solution (32 μL) and acetic acid (27 μL) were added at 0°C, and the resulting mixture was stirred at 0°C for 25 hours. Methanol (450 μL) was added to the obtained reaction mixture (50 μL), to provide a measurement sample. The obtained measurement sample was subjected to HPLC measurement by the method described above. As a result, the bound-drug purity was found to be 94.5%.

Test Example 7

Evaluation of Bound-Drug Purity of P-THP (Example 7) Using Hydroxylamine, Acetic Acid, and Methanol:

To a solution of the P-THP (10 mg) obtained by Example 7 in methanol (160 μL), 50% by weight aqueous hydroxylamine solution (32 μL) and acetic acid (27 μL) were added at 0°C, and the resulting mixture was stirred at 0°C for 25 hours. Methanol (450 μL) was added to the obtained reaction mixture (50 μL), to provide a measurement sample. The obtained measurement sample was subjected to HPLC measurement by the method described above. As a result, the bound-drug purity was found to be 98.9%.

Test Example 8

Evaluation of Bound-Drug Purity of P-THP (Example 7) Using Acetohydrazide, Acetic Acid, and Methanol:

To a solution of the P-THP (10 mg) obtained by Example 7 in methanol (160 μL), acetohydrazide (35 mg) and acetic acid (27 μL) were added at 0°C, and the resulting mixture was stirred at 0°C for 20 hours. Methanol (450 μL) was added to the obtained reaction mixture (50 μL), to provide a measurement sample. The obtained measurement sample was subjected to HPLC measurement by the method described above. As a result, the bound-drug purity was found to be 97.7%.

Test Example 9

Evaluation of Bound-Drug Purity of P-THP (Example 7) Using Propanohydrazide, Acetic Acid, and Methanol:

To a solution of the P-THP (10 mg) obtained by Example 7 in methanol (160 μL), propanohydrazide (42 mg) and acetic acid (27 μL) were added at 0°C, and the resulting mixture was stirred at 0°C for 20 hours. Methanol (450 μL) was added to the obtained reaction mixture (50 μL), to provide a measurement sample. The obtained measurement sample was subjected to HPLC measurement by the method described above. As a result, the bound-drug purity was found to be 96.0%.

Test Example 10

Evaluation of Bound-Drug Purity of P-THP (Example 7) Using Butyrohydrazide, Acetic Acid, and Methanol:

To a solution of the P-THP (10 mg) obtained by Example 7 in methanol (160 μL), butyrohydrazide (49 mg) and acetic acid (27 μL) were added at 0°C, and the resulting mixture was stirred at 0°C for 20 hours. Methanol (450 μL) was added to the obtained reaction mixture (50 μL), to provide a measurement sample. The obtained measurement sample was subjected to HPLC measurement by the method described above. As a result, the bound-drug purity was found to be 96.8%.

Test Example 11

Evaluation of Bound-Drug Purity of P-THP (Example 7) Using 3-Methylbutanohydrazide, Acetic Acid, and Methanol:

To a solution of the P-THP (10 mg) obtained by Example 7 in methanol (160 μL), 3-methylbutanohydrazide (56 mg) and acetic acid (27 μL) were added at 0°C, and the resulting mixture was stirred at 0°C for 20 hours. Methanol (450 μL) was added to the obtained reaction mixture (50 μL), to provide a measurement sample. The obtained measurement sample was subjected to HPLC measurement by the method described above. As a result, the bound-drug purity was found to be 96.6%.

The results of Test Examples 6 to 11 are shown in Table 2.

[Table 2]

| | Complex | Type and amount of use (weight) of nitrogen-containing nucleophile | Type and amount of use (weight) of acid | Type and amount of use (weight) of polar solvent | Reaction temperature (°C) | Bound-drug purity (%) |
|---|---|---|---|---|---|---|
| Test Example 6 | P-THP (Comparative Example 1) | Hydroxylamine 3.2 times the weight | Acetic acid 2.8 times the weight | Methanol 13 times the weight | 0 | 94.5 |
| Test Example 7 | P-THP (Example 7) | Hydroxylamine 3.2 times the weight | Acetic acid 2.8 times the weight | Methanol 13 times the weight | 0 | 98.9 |
| Test Example 8 | P-THP (Example 7) | Acetohydrazide 3.5 times the weight | Acetic acid 2.8 times the weight | Methanol 13 times the weight | 0 | 97.7 |
| Test Example 9 | P-THP (Example 7) | Propanohydrazide 4.2 times the weight | Acetic acid 2.8 times the weight | Methanol 13 times the weight | 0 | 96.0 |
| Test Example 10 | P-THP (Example 7) | Butyrohydrazide 4.9 times the weight | Acetic acid 2.8 times the weight | Methanol 13 times the weight | 0 | 96.8 |
| Test Example 11 | P-THP (Example 7) | 3-Methylbutanohydrazide 5.6 times the weight | Acetic acid 2.8 times the weight | Methanol 13 times the weight | 0 | 96.6 |

As shown in Table 2, as a result of subjecting the P-THP obtained by Comparative Example 1 to the reaction conditions of Test Example 4, nucleophilic substitution reaction for the complex efficiently proceeded, but the P-TIIP did

not show a bound-drug purity of not less than 95.0%, so that the P-THP produced by the conventional method was found to have a problem in the bound-drug purity (Test Example 6). In contrast, when the P-THP obtained by Example 7 was subjected to the same conditions as in Test Example 6, the P-THP was found to show a bound-drug purity of as high as 98.9% (Test Example 7). Further, the P-THP obtained by Example 7 was found to show good bound-drug purities also in cases where various carboxylic acid hydrazides were used to evaluate the bound-drug purity (Test Examples 8 to 11).

Example 2

Method of Producing P-THP under Following Conditions: *N*-(2-Hydroxypropyl)Methacrylamide Polymer, 8.8 Times Weight; Acetic Acid, 40 Molar Equivalents; Methanol, 24 Times Weight; Reaction Temperature, 1 to 2°C; Reaction Time, 15 Hours:

To a solution of an *N*-(2-hydroxypropyl)methacrylamide polymer (526 mg, manufactured by the Institute of Macromolecular Chemistry, Czech Academy of Sciences) in methanol (1.8 mL), THP (60.0 mg, manufactured by MicroBiopharm Japan Co., Ltd.) and acetic acid (219 μL) were added, and the resulting mixture was stirred at 1 to 2°C for 15 hours, followed by adding methanol (3.2 mL) to the reaction mixture. The reaction mixture obtained was added to ethyl acetate (21 mL), and the resulting mixture was stirred at 33 to 34°C for 24 hours. After collecting the precipitated solid by filtration, the solid was dried at 31 to 32°C under reduced pressure, to obtain P-THP (yield, 562 mg; percent yield, 96.1%).

Example 3

Method of Producing P-THP under Following Conditions: *N*-(2-Hydroxypropyl)Methacrylamide Polymer, 9.1 Times Weight; Acetic Acid, 32 Molar Equivalents; Methanol, 58 Times Weight; Reaction Temperature, 1 to 2°C; Reaction Time, 40 Hours:

To a solution of an *N*-(2-hydroxypropyl)methacrylamide polymer (200 mg, manufactured by the Institute of Macromolecular Chemistry, Czech Academy of Sciences) in methanol (1.6 mL), THP (22.0 mg, manufactured by MicroBiopharm Japan Co., Ltd.) and acetic acid (65 μL) were added, and the resulting mixture was stirred at 1 to 2°C for 40 hours, followed by adding methanol (0.3 mL) to the reaction mixture. The reaction mixture obtained was added to ethyl acetate (7.7 mL), and the resulting mixture was stirred at 21 to 22°C for 2 hours, followed by collecting the precipitated solid by filtration. A solution of the obtained solid in methanol (1.4 mL) was added to ethyl acetate (5.7 mL), and the resulting mixture was stirred at 22 to 23°C for 2 hours. After collecting the precipitated solid by filtration, the solid was dried at 30 to 31°C under reduced pressure, to obtain P-THP (yield, 205 mg; percent yield, 92.8%).

Example 4

Method of Producing P-THP under Following Conditions: *N*-(2-Hydroxypropyl)Methacrylamide Polymer, 8.8 Times Weight; Acetic Acid, 40 Molar Equivalents; Methanol, 24 Times Weight; Reaction Temperature, 1 to 5°C; Reaction Time, 16 Hours:

To a solution of an *N*-(2-hydroxypropyl)methacrylamide polymer (3.16 g, manufactured by Chemicalsoft Co., Ltd.) in methanol (10.8 mL), THP (360 mg, manufactured by MicroBiopharm Japan Co., Ltd.) and acetic acid (1.31 mL) were added, and the resulting mixture was stirred at 1 to 5°C for 16 hours, followed by adding methanol (19.2 mL) to the reaction mixture. The reaction mixture obtained was added to ethyl acetate (126 mL), and the resulting mixture was stirred at 19 to 22°C for 2 hours, followed by collecting the precipitated solid by filtration. A solution of the obtained solid in methanol (24.0 mL) was added to ethyl acetate (96.0 mL), and the resulting mixture was stirred at 17 to 21°C for 2 hours. After collecting the precipitated solid by filtration, the solid was dried at 30 to 37°C under reduced pressure, to obtain P-THP (yield, 3.43 g; percent yield, 97.8%).

Example 5

Method of Producing P-THP under Following Conditions: *N*-(2-Hydroxypropyl)Methacrylamide Polymer, 8.8 Times Weight; Acetic Acid, 100 Molar Equivalents; Methanol, 56 Times Weight; Reaction Temperature, 2 to 4°C; Reaction Time, 15 Hours:

To a solution of an *N*-(2-hydroxypropyl)methacrylamide polymer (3.16 g, manufactured by Chemicalsoft Co.,

Ltd.) in methanol (25.2 mL), THP (360 mg, manufactured by MicroBiopharm Japan Co., Ltd.) and acetic acid (3.28 mL) were added, and the resulting mixture was stirred at 2 to 4°C for 15 hours, followed by adding methanol (2.8 mL) to the reaction mixture. The reaction mixture obtained was added to ethyl acetate (126 mL), and the resulting mixture was stirred at 21 to 22°C for 2 hours, followed by collecting the precipitated solid by filtration. A solution of the obtained solid in methanol (24.0 mL) was added to ethyl acetate (96.0 mL), and the resulting mixture was stirred at 21 to 24°C for 2 hours. After collecting the precipitated solid by filtration, the solid was dried at 35 to 42°C under reduced pressure, to obtain P-THP (yield, 3.48 g; percent yield, 99.3%).

Example 6

Method of Producing P-THP under Following Conditions: *N*-(2-Hydroxypropyl)Methacrylamide Polymer, 8.8 Times Weight; Acetic Acid, 40 Molar Equivalents; Methanol, 24 Times Weight; Reaction Temperature, -10 to -9°C; Reaction Time, 38 Hours:

To a solution of an *N*-(2-hydroxypropyl)methacrylamide polymer (526 mg, manufactured by the Institute of Macromolecular Chemistry, Czech Academy of Sciences) in methanol (1.8 mL), THP (60.0 mg, manufactured by MicroBiopharm Japan Co., Ltd.) and acetic acid (219 μL) were added, and the resulting mixture was stirred at -10 to -9°C for 38 hours, followed by adding methanol (3.2 mL) to the reaction mixture. The reaction mixture obtained was added to ethyl acetate (21 mL), and the resulting mixture was stirred at 1 to 2°C for 1 hour, followed by collecting the precipitated solid by filtration. A solution of the obtained solid in methanol (4.0 mL) was added to ethyl acetate (16.0 mL), and the resulting mixture was stirred at 1 to 5°C for 1 hour. After collecting the precipitated solid by filtration, the solid was dried at 31 to 32°C under reduced pressure, to obtain P-THP (yield, 541 mg; percent yield, 92.6%).

Example 7

Method of Producing P-THP under Following Conditions: *N*-(2-Hydroxypropyl)Methacrylamide Polymer, 8.8 Times Weight; Acetic Acid, 40 Molar Equivalents; Methanol, 24 Times Weight; Reaction Temperature, -30 to -29°C; Reaction Time, 112 Hours:

To a solution of an *N*-(2-hydroxypropyl)methacrylamide polymer (526 mg, manufactured by the Institute of Macromolecular Chemistry, Czech Academy of Sciences) in methanol (1.8 mL), THP (60.0 mg, manufactured by MicroBiopharm Japan Co., Ltd.) and acetic acid (219 μL) were added, and the resulting mixture was stirred at -30 to -29°C for 112 hours, followed by adding methanol (3.2 mL) to the reaction mixture. The reaction mixture obtained was added to ethyl acetate (21 mL), and the resulting mixture was stirred at 22 to 23°C for 0.5 hour. After collecting the precipitated solid by filtration, the solid was dried at 32 to 33°C under reduced pressure, to obtain P-TIIP (yield, 548 mg; percent yield, 93.7%).

Example 8

Method of Producing P-THP under Following Conditions: *N*-(2-Hydroxypropyl)Methacrylamide Polymer, 8.8 Times Weight; Acetic Acid, 40 Molar Equivalents; Methanol, 24 Times Weight; Reaction Temperature, 2 to 5°C; Reaction Time, 15 Hours:

To a solution of an *N*-(2-hydroxypropyl)methacrylamide polymer (13.2 g, manufactured by the Institute of Macromolecular Chemistry, Czech Academy of Sciences) in methanol (45.0 mL), THP (1.50 g, manufactured by MicroBiopharm Japan Co., Ltd.) and acetic acid (5.47 mL) were added, and the resulting mixture was stirred at 2 to 5°C for 15 hours, followed by adding methanol (79.5 mL) to the reaction mixture. The reaction mixture obtained was added to ethyl acetate (525 mL), and the resulting mixture was stirred at 20 to 22°C for 1 hour, followed by collecting the precipitated solid by filtration. A solution of the obtained solid in methanol (97.5 mL) was added to ethyl acetate (390 mL), and the resulting mixture was stirred at 22 to 23°C for 1 hour. After collecting the precipitated solid by filtration, the solid was dried at 31 to 32°C under reduced pressure, to obtain P-THP (yield, 14.2 g; percent yield, 97.4%).

Example 9

Method of Producing P-DOX Hydrochloride under Following Conditions: *N*-(2-Hydroxypropyl)Methacrylamide Polymer, 9.8 Times Weight; Acetic Acid, 100 Molar Equivalents; Methanol, 62 Times Weight; Reaction Temperature, 1 to 4°C; Reaction Time, 46 Hours:

To a solution of an *N*-(2-hydroxypropyl)methacrylamide polymer (200 mg, manufactured by the Institute of Macromolecular Chemistry, Czech Academy of Sciences) in methanol (1.6 mL), DOX hydrochloride (20.3 mg, manufactured by MedKoo Biosciences, Inc.) and acetic acid (200 μL) were added, and the resulting mixture was stirred at 1 to 4°C for 46 hours, followed by adding methanol (0.3 mL) to the reaction mixture. The reaction mixture obtained was added to ethyl acetate (7.7 mL), and the resulting mixture was stirred at 21 to 22°C for 1 hour. After collecting the precipitated solid by filtration, the solid was dried at 32 to 33°C under reduced pressure, to obtain P-DOX hydrochloride (yield, 219 mg; percent yield, 99.7%).

Comparative Example 1

Method of Producing P-THP under Following Conditions: *N*-(2-Hydroxypropyl)Methacrylamide Polymer, 9.1 Times Weight; Acetic Acid, 32 Molar Equivalents; Methanol, 58 Times Weight; Reaction Temperature, 29 to 30°C; Reaction Time, 15 Hours (See Temperature Described in Patent Document 1 and Method Described in Non-Patent Document 1):

To a solution of an *N*-(2-hydroxypropyl)methacrylamide polymer (200 mg, manufactured by the Institute of Macromolecular Chemistry, Czech Academy of Sciences) in methanol (1.6 mL), THP (22.0 mg, manufactured by Micro-Biopharm Japan Co., Ltd.) and acetic acid (65 μL) were added, and the resulting mixture was stirred under dark condition at 29 to 30°C for 15 hours, followed by adding methanol (0.3 mL) to the reaction mixture. The reaction mixture obtained was added to ethyl acetate (7.7 mL), and the resulting mixture was stirred at 29 to 32°C for 24 hours. After collecting the precipitated solid by filtration, the solid was dried at 31 to 34°C under reduced pressure, to obtain P-THP (yield, 209 mg; percent yield, 94.3%).

Comparative Example 2

Method of Producing P-THP under Following Conditions: *N*-(2-Hydroxypropyl)Methacrylamide Polymer, 8.8 Times Weight; Acetic Acid, 40 Molar Equivalents; Methanol, 56 Times Weight; Reaction Temperature, 34 to 35°C; Reaction Time, 17 Hours:

To a solution of an *N*-(2-hydroxypropyl)methacrylamide polymer (3.16 g, manufactured by the Institute of Macromolecular Chemistry, Czech Academy of Sciences) in methanol (25.2 mL), THP (360 mg, manufactured by MicroBiopharm Japan Co., Ltd.) and acetic acid (1.31 mL) were added, and the resulting mixture was stirred at 34 to 35°C for 17 hours, followed by adding methanol (4.8 mL) to the reaction mixture. The reaction mixture obtained was added to ethyl acetate (126 mL), and the resulting mixture was stirred at 19 to 23°C for 1 hour, followed by collecting the precipitated solid by filtration. A solution of the obtained solid in methanol (24.0 mL) was added to ethyl acetate (96.0 mL), and the resulting mixture was stirred at 22 to 23°C for 1 hour. After collecting the precipitated solid by filtration, the solid was dried at 31 to 35°C under reduced pressure, to obtain P-THP (yield, 3.47 g; percent yield, 98.9%).

Example 10

Method of Producing P-THP under Following Conditions: *N*-(2-Hydroxypropyl)Methacrylamide Polymer, 24.0 Times Weight; Acetic Acid, 100 Molar Equivalents; Methanol, 56 Times Weight; Reaction Temperature, 4 to 7°C; Reaction Time, 20 Hours:

To a solution of an *N*-(2-hydroxypropyl)methacrylamide polymer (1.20 g, manufactured by the Institute of Macromolecular Chemistry, Czech Academy of Sciences) in methanol (3.5 mL), THP (50 mg, manufactured by MicroBiopharm Japan Co., Ltd.) and acetic acid (0.46 mL) were added, and the resulting mixture was stirred at 4 to 7°C for 20 hours, followed by adding methanol (2.0 mL) to the reaction mixture. The reaction mixture obtained was added to ethyl acetate (25 mL), and the resulting mixture was stirred at 22 to 23°C for 1 hour, followed by collecting the precipitated solid by filtration. A solution of the obtained solid in methanol (10.0 mL) was added to ethyl acetate (40.0 mL), and the resulting mixture was stirred at 21 to 23°C for 1 hour. After collecting the precipitated solid by filtration, the solid was dried at 23 to 24°C under reduced pressure, to obtain P-THP (yield, 1.11 g; percent yield, 89.4%).

Example 11

Method of Producing P-THP under Following Conditions: *N*-(2-Hydroxypropyl)Methacrylamide Polymer, 13.3 Times Weight; Acetic Acid, 100 Molar Equivalents; Methanol, 56 Times Weight; Reaction Temperature, 2 to 4°C; Reaction Time, 21 Hours:

To a solution of an *N*-(2-hydroxypropyl)methacrylamide polymer (0.66 g, manufactured by the Institute of Macromolecular Chemistry, Czech Academy of Sciences) in methanol (3.5 mL), THP (50 mg, manufactured by MicroBiopharm Japan Co., Ltd.) and acetic acid (0.46 mL) were added, and the resulting mixture was stirred at 2 to 4°C for 21 hours, followed by adding methanol (2.0 mL) to the reaction mixture. The reaction mixture obtained was added to ethyl acetate (25 mL), and the resulting mixture was stirred at 22 to 23°C for 1 hour, followed by collecting the precipitated solid by filtration and drying the solid at 22 to 23°C under reduced pressure, to obtain P-THP (yield, 0.70 g; percent yield, 98.4%).

Example 12

Method of Producing P-THP under Following Conditions: *N*-(2-Hydroxypropyl)Methacrylamide Polymer, 8.8 Times Weight; Acetic Acid, 40 Molar Equivalents; Methanol, 24 Times Weight; Reaction Temperature, 1 to 5°C; Reaction Time, 19 Hours:

To a solution of an *N*-(2-hydroxypropyl)methacrylamide polymer (122.8 g, manufactured by the Institute of Macromolecular Chemistry, Czech Academy of Sciences) in methanol (420 mL), THP (14.0 g, manufactured by MicroBiopharm Japan Co., Ltd.) and acetic acid (51.0 mL) were added, and the resulting mixture was stirred at 1 to 5°C for 19 hours, followed by adding methanol (742 mL) to the reaction mixture. The reaction mixture obtained was added to ethyl acetate (4900 mL), and the resulting mixture was stirred at 18 to 20°C for 1 hour, followed by collecting the precipitated solid by filtration. A solution of the obtained solid in methanol (910 mL) was added to ethyl acetate (3640 mL), and the resulting mixture was stirred at 18 to 20°C for 1 hour. After collecting the precipitated solid by filtration, the solid was dried at 16 to 20°C under reduced pressure, to obtain P-THP (yield, 130.2 g; percent yield, 95.4%).

Example 13

Method of Producing P-THP under Following Conditions: *N*-(2-Hydroxypropyl)Methacrylamide Polymer, 6.7 Times Weight; Acetic Acid, 60 Molar Equivalents; Methanol, 16 Times Weight; Reaction Temperature, 4 to 7°C; Reaction Time, 22 Hours:

To a solution of an *N*-(2-hydroxypropyl)methacrylamide polymer (0.67 g, manufactured by the Institute of Macromolecular Chemistry, Czech Academy of Sciences) in methanol (2.0 mL), THP (100 mg, manufactured by MicroBiopharm Japan Co., Ltd.) and acetic acid (0.55 mL) were added, and the resulting mixture was stirred at 4 to 7°C for 22 hours, followed by adding methanol (2.0 mL) to the reaction mixture. The reaction mixture obtained was added to ethyl acetate (25 mL), and the resulting mixture was stirred at 22 to 23°C for 1 hour, followed by collecting the precipitated solid by filtration and drying the solid at 21 to 22°C under reduced pressure, to obtain P-THP (yield, 0.76 g; percent yield, 98.6%).

Example 14

Method of Producing P-THP under Following Conditions: *N*-(2-Ilydroxypropyl)Methacrylamide Polymer, 5.0 Times Weight; Acetic Acid, 60 Molar Equivalents; Methanol, 16 Times Weight; Reaction Temperature, 1 to 5°C; Reaction Time, 20 Hours:

To a solution of an *N*-(2-hydroxypropyl)methacrylamide polymer (3.00 g, manufactured by the Institute of Macromolecular Chemistry, Czech Academy of Sciences) in methanol (12.0 mL), THP (600 mg, manufactured by MicroBiopharm Japan Co., Ltd.) and acetic acid (3.28 mL) were added, and the resulting mixture was stirred at 1 to 5°C for 20 hours, followed by adding methanol (15.0 mL) to the reaction mixture. The reaction mixture obtained was added to ethyl acetate (120 mL), and the resulting mixture was stirred at 19 to 21°C for 0.5 hour, followed by collecting the precipitated solid by filtration. A solution of the obtained solid in methanol (27.0 mL) was added to ethyl acetate (120 mL), and the resulting mixture was stirred at 19 to 23°C for 1 hour. After collecting the precipitated solid by filtration, the solid was dried at 23 to 24°C under reduced pressure, to obtain P-THP (yield, 3.37 g; percent yield, 93.5%).

Example 15

Method of Producing P-THP under Following Conditions: *N*-(2-Hydroxypropyl)Methacrylamide Polymer, 4.0 Times Weight; Acetic Acid, 40 Molar Equivalents; Methanol, 16 Times Weight; Reaction Temperature, 1 to 4°C; Reaction Time, 37 Hours:

To a solution of an *N*-(2-hydroxypropyl)methacrylamide polymer (200 mg, manufactured by the Institute of Macromolecular Chemistry, Czech Academy of Sciences) in methanol (1.0 mL), THP (50 mg, manufactured by Micro-Biopharm Japan Co., Ltd.) and acetic acid (0.18 mL) were added, and the resulting mixture was stirred at 1 to 4°C for 37 hours, followed by adding methanol (0.8 mL) to the reaction mixture. The reaction mixture obtained was added to ethyl acetate (10 mL), and the resulting mixture was stirred at 1 to 5°C for 1 hour, followed by collecting the precipitated solid by filtration. A solution of the obtained solid in methanol (3.0 mL) was added to ethyl acetate (15 mL), and the resulting mixture was stirred at 22 to 23°C for 0.5 hour. After collecting the precipitated solid by filtration, the solid was dried at 23 to 24°C under reduced pressure, to obtain P-THP (yield, 197 mg; percent yield, 78.9%).

The results of Examples 2 to 15, Comparative Example 1, and Comparative Example 2 are shown in Table 3.

[Table 3]

| | Type and amount of use (mg) of anthracycline drug | Type[1)] and amount of use (weight) of *N*-(2-hydroxypropyl) methacrylamide polymer | Type and amount of use (molar equivalents) of protonic acid | Type and amount of use (weight) of polar solvent | Reaction temperature (°C) | Reaction time (hours) | Bound-drug purity (%) | Amount of carried bound drug (wt%) | Weight average molecular weight (Mw) |
|---|---|---|---|---|---|---|---|---|---|
| Example 2 | THP 60.0 | (P) 8.8 times | Acetic acid 40 | Methanol 24 times | 1 to 2 | 15 | 98.2 | 9.7 | 38000 |
| Example 3 | THP 22.0 | (P) 9.1 times | Acetic acid 32 | Methanol 58 times | 1 to 2 | 40 | 98.5 | 9.5 | 40000 |
| Example 4 | THP 360 | (Q) 8.8 times | Acetic acid 40 | Methanol 24 times | 1 to 5 | 16 | 98.9 | 9.4 | 36000 |
| Example 5 | THP 360 | (Q) 8.8 times | Acetic acid 100 | Methanol 56 times | 2 to 4 | 15 | 98.5 | 9.3 | 35000 |
| Example 6 | THP 60.0 | (P) 8.8 times | Acetic acid 40 | Methanol 24 times | -10 to -9 | 38 | 99.0 | 10.5 | 40000 |
| Example 7 | THP 60.0 | (P) 8.8 times | Acetic acid 40 | Methanol 24 times | -30 to -29 | 112 | 99.0 | 10.2 | 40000 |
| Example 8 | THP 1500 | (P) 8.8 times | Acetic acid 40 | Methanol 24 times | 2 to 5 | 15 | 98.9 | 9.7 | 39000 |
| Example 9 | DOX hydrochloride 20.3 | (P) 9.8 times | Acetic acid 100 | Methanol 62 times | 1 to 4 | 46 | 99.5 | 8.6 | 35000 |
| Comparative Example 1 | THP 22.0 | (P) 9.1 times | Acetic acid 32 | Methanol 58 times | 29 to 30 | 15 | 94.5 | 8.5 | 40000 |
| Comparative Example 2 | THP 360 | (P) 8.8 times | Acetic acid 40 | Methanol 56 times | 34 to 35 | 17 | 92.6 | 9.0 | 39000 |
| Example 10 | THP 50.0 | (P) 24.0 times | Acetic acid 100 | Methanol 56 times | 4 to 7 | 20 | 99.0 | 4.0 | 40000 |
| Example 11 | THP 50.0 | (P) 13.3 times | Acetic acid 100 | Methanol 56 times | 2 to 4 | 21 | 99.0 | 7.3 | 40000 |
| Example 12 | THP 14000 | (P) 8.8 times | Acetic acid 40 | Methanol | 1 to 5 | 19 | 98.9 | 10.3 | 36000 |

(continued)

| | Type and amount of use (mg) of anthracycline drug | Type[1)] and amount of use (weight) of *N*-(2-hydroxypropyl) methacrylamide polymer | Type and amount of use (molar equivalents) of protonic acid | Type and amount of use (weight) of polar solvent | Reaction temperature (°C) | Reaction time (hours) | Bound-drug purity (%) | Amount of carried bound drug (wt%) | Weight average molecular weight (Mw) |
|---|---|---|---|---|---|---|---|---|---|
| | | | | 24 times | | | | | |
| Example 13 | THP 100 | (P) 6.7 times | Acetic acid 60 | Methanol 16 times | 4 to 7 | 22 | 98.8 | 12.4 | 36000 |
| Example 14 | THP 600 | (P) 5.0 times | Acetic acid 60 | Methanol 16 times | 1 to 5 | 20 | 98.5 | 16.1 | 39000 |
| Example 15 | THP 50.0 | (P) 4.0 times | Acetic acid 40 | Methanol 16 times | 1 to 4 | 37 | 98.3 | 18.4 | 37000 |

1) (P) manufactured by the Institute of Macromolecular Chemistry, Czech Academy of Sciences; (Q) manufactured by Chemicalsoft Co., Ltd.

As a result of evaluating the complexes obtained by Examples 2 to 15, Comparative Example 1, and Comparative Example 2 by the evaluation method using hydroxylamine, acetic acid, and methanol, it was found, as shown in Table 3, that a desired product having a higher bound-drug purity than those of Comparative Examples 1 and 2 can be obtained by setting the reaction temperature to not more than 10°C (Examples 2 to 15). Further, also in the cases where a different type of anthracycline drug or a different type of *N*-(2-hydroxypropyl)methacrylamide polymer was used, good bound-drug purities were obtained (Examples 4, 5, and 9), and, also in the cases where a different amount of *N*-(2-hydroxypropyl)methacrylamide polymer, a different molar equivalence of acetic acid, and/or a different amount of methanol were used, bound-drug purities of not less than 98.0% were found (Examples 3, 5, and 9). Thus, the reaction temperature was found to be important for the improvement of the bound-drug purity. Further, it was found that the reaction efficiently proceeds also in cases where the reaction temperature is about -10°C or -30°C (Examples 6 and 7), and that scale-up production is possible (Example 8).

Further, it was found that, even in cases where the amount of the *N*-(2-hydroxypropyl)methacrylamide polymer relative to the anthracycline drug is largely changed, the amount of the carried bound drug can be changed while maintaining a bound-drug purity of not less than 98.0%, by setting the reaction temperature to not more than 10°C (Examples 10 to 15). It was also found that the use of an even higher reaction temperature than in Comparative Example 1 results in a further decrease in the bound-drug purity (Comparative Example 2).

Example 16

Evaluation of Storage Stability under Airtight Conditions at 60°C (Changes in Weight Average Molecular Weight over Time in Short-Term Stress Test):

Each of the complexes (5 mg) obtained in Comparative Example 1 and Examples 12 to 15 was weighed, and placed in an HPLC vial. After closing the lid, the vial was left to stand in an oven at 60°C. The vial was removed from the oven at week 2 or week 4, and then the weight average molecular weight of the complex was evaluated. Table 4 shows the results of the evaluation of the weight average molecular weight at week 2 and week 4, wherein week 0 corresponds to the time when the evaluation of the storage stability has been started.

[Table 4]

| | Bound-drug purity (%) | Amount of carried bound drug (wt%) | Weight average molecular weight (Mw) | | |
|---|---|---|---|---|---|
| | | | Week 0 | Week 2 | Week 4 |
| Comparative Example 1 | 94.5 | 8.5 | 40000 | 102000 | 1000000 |
| Example 12 | 98.9 | 10.3 | 36000 | 57000 | 137000 |
| Example 13 | 98.8 | 12.4 | 36000 | 54000 | 130000 |
| Example 14 | 98.5 | 16.1 | 39000 | 50000 | 89000 |
| Example 15 | 98.3 | 18.4 | 37000 | 40000 | 58000 |

As shown in Table 4, based on comparison of the changes in the weight average molecular weight over time and the bound-drug purity, it was found that, in cases where the bound-drug purity is less than 95.0%, the weight average molecular weight remarkably increases, that is, the storage stability is low. In contrast, it was found that, in cases where the bound-drug purity is not less than 95.0%, the phenomenon that the molecular weight of the complex increases can be remarkably suppressed, and high storage stability can be achieved (Comparative Example 1, Example 12). Further, based on comparison of the changes in the weight average molecular weight over time and the amount of the carried bound drug, it was found that, by increasing the amount of the carried bound drug to not less than 15 wt%, the phenomenon that the molecular weight of the complex increases can be further suppressed, and even higher storage stability can be achieved (Examples 12 to 15).

INDUSTRIAL APPLICABILITY

By the method of evaluating the purity of the drug contained in the Complex (1) or the pharmaceutically acceptable salt thereof according to the present invention, whether or not the bound-drug purity satisfies the purity standard for THP or DOX hydrochloride described in the Japanese Pharmacopoeia can be evaluated. Further, by the production method of the present invention, a Complex (I) having a high purity that has not been conventionally achieved can be

produced.

## Claims

1. A method of evaluating the purity of a drug contained in a complex of General Formula (I):

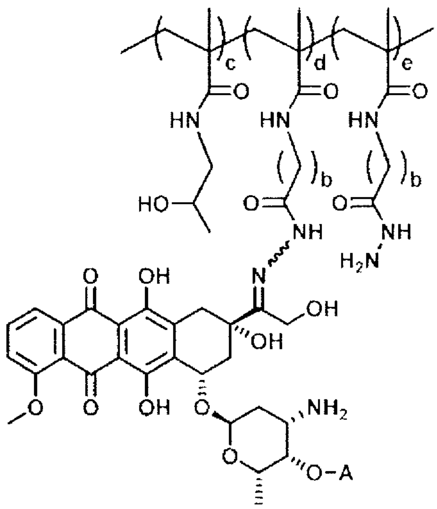

(I)

[wherein A is a hydrogen atom or (*R*)-tetrahydro-2*H*-pyran-2-yl; b, c, d, and e each independently are a positive integer; and the bond indicated by a wavy line represents that the complex has either *E* or *Z* configuration] or a pharmaceutically acceptable salt thereof, the method comprising:

a reaction step of reacting the complex of General Formula (I) or the pharmaceutically acceptable salt thereof with at least one nitrogen-containing nucleophile selected from the group consisting of hydroxylamine, *O*-alkylhydroxylamine, and carboxylic acid hydrazide in the presence of a protonic acid in a polar solvent; and
an evaluation step of evaluating the purity of the reaction mixture obtained by the reaction step, by high-performance liquid chromatography.

2. The method according to claim 1,
wherein

the polar solvent is an alcoholic solvent;
the nitrogen-containing nucleophile is at least one selected from the group consisting of hydroxylamine and carboxylic acid hydrazide; and
the protonic acid is a carboxylic acid.

3. The method according to claim 1 or 2,
wherein

the polar solvent is methanol;
the nitrogen-containing nucleophile is at least one selected from the group consisting of hydroxylamine, acctohydrazide, propanohydrazide, butyrohydrazide, and 3-methylbutanohydrazide; and
the protonic acid is acetic acid.

4. The method according to any one of claims 1 to 3, wherein in the General Formula (I), b is an integer of 1 to 10; c is an integer of 30 to 500; d is an integer of 1 to 50; and e is an integer of 1 to 50.

5. A complex of General Formula (I):

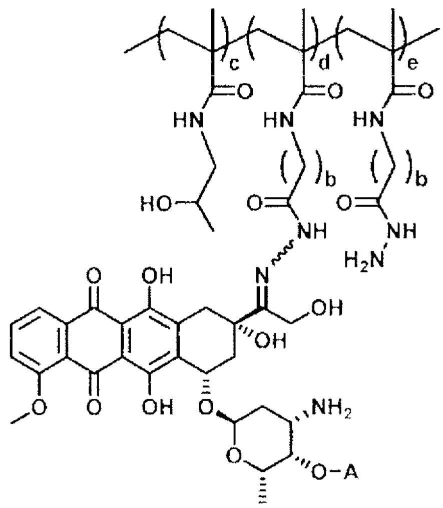

(I)

[wherein A is a hydrogen atom or (*R*)-tetrahydro-2*H*-pyran-2-yl; b, c, d, and e each independently are a positive integer; and the bond indicated by a wavy line represents that the complex has either *E* or *Z* configuration] or a pharmaceutically acceptable salt thereof, wherein a drug contained in the complex of General Formula (1) or the pharmaceutically acceptable salt thereof has a purity of not less than 95.0% as evaluated by the method according to any one of claims 1 to 4.

**6.** The complex or the pharmaceutically acceptable salt thereof according to claim 5, wherein

A is (*R*)-tetrahydro-2*H*-pyran-2-yl; and
b is 5.

**7.** A method of producing a complex of General Formula (1):

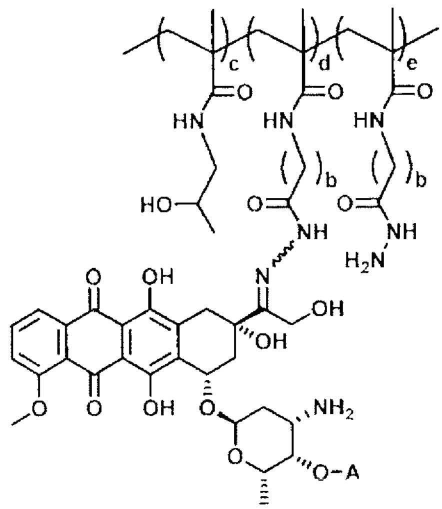

(I)

or a pharmaceutically acceptable salt thereof, the method comprising a reaction step of reacting an anthracycline drug of General Formula (II):

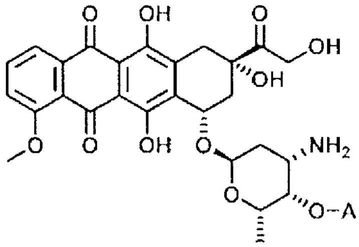

(II)

with an *N*-(2-hydroxypropyl)methacrylamide polymer of General Formula (III):

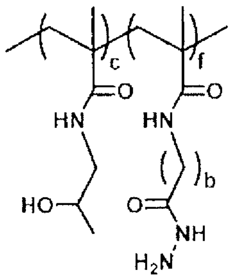

(III)

in the presence of a protonic acid in a polar solvent at not more than 10°C, to obtain the complex of General Formula (I) or the pharmaceutically acceptable salt thereof [wherein in Formula (1) and Formula (II), A is a hydrogen atom or (*R*)-tetrahydro-2*H*-pyran-2-yl; and, in Formula (I) and Formula (111), b, c, d, e, and f each independently are a positive integer, and the bond indicated by a wavy line represents that the complex has either *E* or *Z* configuration].

**8.** The method according to claim 7, wherein

the polar solvent is methanol;
the protonic acid is acetic acid; and
the reaction temperature during the reaction step is -30°C to 10°C.

**9.** The method according to claim 7 or 8, wherein in the General Formula (I) and the General Formula (III), b is an integer of 1 to 10; c is an integer of 30 to 500; d is an integer of 1 to 50; e is an integer of 1 to 50; and f is the sum of d and e.

## INTERNATIONAL SEARCH REPORT

International application No.
**PCT/JP2021/028274**

**A. CLASSIFICATION OF SUBJECT MATTER**

***C08F 20/56***(2006.01)i; ***A61K 47/58***(2017.01)i; ***G01N 30/06***(2006.01)i; ***G01N 30/88***(2006.01)i
FI: G01N30/06 E; C08F20/56; G01N30/88 P; A61K47/58

According to International Patent Classification (IPC) or to both national classification and IPC

**B. FIELDS SEARCHED**

Minimum documentation searched (classification system followed by classification symbols)

C08F20/56; A61K47/58; G01N30/06; G01N30/88

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| A<br><br><br><br><br><br>X | ETRYCH, T. et al., Comparison of the pharmacological and biological properties of HPMA copolymer-pirarubicin conjugates: A single-chain copolymer conjugate and its biodegradable tandem-diblock copolymer conjugate, European Journal of Pharmaceutical Sciences, 2017, Vol.106, pp.10-19<br>p. 11, right column, line 12 to p. 205, right column, line 1 | 1-4, 7-9<br><br><br><br><br><br>5-6 |
| A<br><br><br><br>X | NAKAMURA, H. et al., Pronounced Cellular Uptake of Pirarubicin versus That of Other Anthracyclines: Comparison of HPMA Copolymer Conjugates of Pirarubicin and Doxorubicin, Molecular Pharmaceutics, 2016, Vol.13, pp.4106-4115<br>p. 4107, right column | 1-4, 7-9<br><br><br><br>5-6 |
| A<br><br><br>X | WO 2017/191843 A1 (BIODYNAMICS LABORATORY INC.) 09 November 2017 (2017-11-09)<br>paragraphs [0015]-[0018], table 1, paragraph [0023] | 1-4, 7-9<br><br><br>5-6 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 October 2021** | **19 October 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br><br>Telephone No. |

Form PCT/ISA/210 (second sheet) (January 2015)

**INTERNATIONAL SEARCH REPORT**

International application No.
**PCT/JP2021/028274**

**C. DOCUMENTS CONSIDERED TO BE RELEVANT**

| Category* | Citation of document, with indication, where appropriate, of the relevant passages | Relevant to claim No. |
|---|---|---|
| A | CONFIGLIACCHI, E. et al., HPLC methods for the determination of bound and free doxorubicin, and of bound and free galactosamine, in methacrylamide polymer-drug conjugates, Journal of Pharmaceutical and Biomedical Analysis, 1996, Vol.15, pp. 123-129<br>abstract | 1-9 |
| A | FRAIER, D. et al., A sensitive procedure for the quantitation of free and N-(2-hydroxypropyl)methacrylamide polymer-bound doxorubicin (PK1) and some of its metabolites, 13- dihydrodoxorubicin, 13-dihydrodoxorubicinone and doxorubicinone, in human plasma and urine by reversed-phase HPLC with fluorimetric detection, Journal of Pharmaceutical & Biomedical Analysis, 1995, Vol.13, No. 4/5, pp. 625-633<br>abstract | 1-9 |
| A | WO 2020/105701 A1 (ONO PHARMACEUTICAL CO., LTD.) 28 May 2020 (2020-05-28)<br>paragraphs [0114]-[0122], [0370] | 1-9 |
| A | WO 2008/047948 A1 (NANOCARRIER CO., LTD.) 24 April 2008 (2008-04-24)<br>claims 10-22, application, p. 15, line 13 to p. 16, line 17 | 1-9 |
| P, A | 中村秀明, 高分子効果を基盤とする腫瘍集積性薬剤の開発研究, 薬学雑誌, 01 October 2020, Vol. 140, No. 10, pp. 1243-1249 (NAKAMURA, Hideaki. Development of Tumor-targeting Antitumor Agents Based on Polymer Effect. Yakugaku Zasshi.)<br>fig. 1 | 1-9 |

**INTERNATIONAL SEARCH REPORT**

International application No.
**PCT/JP2021/028274**

**Box No. III Observations where unity of invention is lacking (Continuation of item 3 of first sheet)**

This International Searching Authority found multiple inventions in this international application, as follows:

Document 1: WO 2017/191843 A1 (BIODYNAMICS LABORATORY INC.) 09 November 2017 (2017-11-09)
Document 2: ETRYCH, T. et al., European Journal of Pharmaceutical Sciences, 2017, Vol. 106, pp. 10-19
(Invention 1) Claims 1-4

The invention as in claims 1-4 is "A purity evaluation method for a drug included in a composite shown by general formula (I) or a pharmacologically acceptable salt thereof," and is classified as invention 1.

(Invention 2) Claims 5-6

The invention as in claims 5-6 is "A composite shown by general formula (I) or a pharmacologically acceptable salt thereof."

Claims 5-6 share, with claim 1 classified as invention 1, the technical feature relating to "a composite shown by general formula (I) or a pharmacologically acceptable salt thereof." However, this technical feature does not make a contribution over the prior art in light of the content disclosed in document 1 (paragraph [0023]) and document 2 (Scheme 2), and thus this technical feature cannot be said to be a special technical feature. Furthermore, there are no other identical or corresponding special technical features between these inventions. It should be noted that claim 5 sets forth "when evaluated with the method set forth in any one of claims 1-4, the purity of the drug included in the composite shown by general formula (I) or the pharmacologically acceptable salt thereof is at least 95.0%." However, the configuration of "a composite shown by general formula (I) or a pharmacologically acceptable salt thereof" as a product is not specified by the evaluation method.

Therefore, the invention as in claims 5-6 cannot be classified as invention 1.

The invention as in claims 5-6 is classified as invention 2.

(Invention 3) Claims 7–9

The invention as in claims 7-9 is "A method for manufacturing a composite shown by general formula (I) or a pharmacologically acceptable salt thereof." Claims 7-9 share, with claim 1 classified as invention 1 and claim 5 classified as invention 2, the technical feature relating to "a composite shown by general formula (I) or a pharmacologically acceptable salt thereof." However, this technical feature does not make a contribution over the prior art in light of the content disclosed in document 1 (paragraph [0023]) and document 2 (Scheme 2), and thus this technical feature cannot be said to be a special technical feature. Document 2 also indicates that, in a polar solvent, an N-(2-hydroxypropyl)methacrylamide polymer shown by general formula (III) was caused to react with an anthracycline-based drug shown by general formula (II) in the presence of protonic acid to obtain a composite shown by general formula (I) or a pharmacologically acceptable salt thereof (document 2, page 11, right column). Furthermore, there are no other identical or corresponding special technical features among these inventions.

Therefore, the invention as in claims 7-9 cannot be classified as either invention 1 or 2.

The invention as in claims 7-9 is classified as invention 3.

**INTERNATIONAL SEARCH REPORT**

International application No.
**PCT/JP2021/028274**

**Box No. III Observations where unity of invention is lacking (Continuation of item 3 of first sheet)**

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2021/028274**

| Patent document cited in search report | Publication date (day/month/year) | Patent family member(s) | Publication date (day/month/year) |
|---|---|---|---|
| WO 2017/191843 A1 | 09 November 2017 | US 2019/0142955 A1<br>paragraphs [0033]-[0038], table 1, paragraph [0042]<br>EP 3453390 A1<br>CN 109069472 A<br>KR 10-2019-0005884 A | |
| WO 2020/105701 A1 | 28 May 2020 | (Family: none) | |
| WO 2008/047948 A1 | 24 April 2008 | US 2010/0298495 A1<br>claims 10-22, paragraphs [0101]-[0102]<br>EP 2077293 A1<br>KR 10-2009-0066302 A<br>CN 101528815 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5904602 B
- WO 2017191843 A


### Non-patent literature cited in the description


- **ETRYCH et al.** *European Journal of Pharmaceutical Sciences,* 2017, vol. 106, 10-19
- **FRAIER et al.** *Journal of Pharmaceutical and Biomedical Analysis,* 1995, vol. 13, 625-633
- **SUMIE YOSHIOKA.** Stability of Medicines. Nankodo Co., Ltd, 1995, 142